# EUROPEAN PATENT SPECIFICATION

(11) **EP 3 167 046 B1**
(45) Date of publication and mention of the grant of the patent: **25.11.2026**
(21) Application number: 15819508.1
(22) Date of filing: 07.07.2015
(51) Int. Cl.: A61K 35/12, A61K 35/17, A61K 35/28, C12N 5/00, C12N 9/10, A01N 1/125

(54) **METHOD OF CRYOPRESERVATION OF FUCOSYLATED CELLS FOR THERAPEUTIC USE**
VERFAHREN ZUR KRYOKONSERVIERUNG VON FUCOSYLIERTEN ZELLEN ZUR THERAPEUTISCHEN VERWENDUNG
METHODE DE CRYOPRÉSERVATION DE CELLULES FUCOSYLÉES À USAGE THÉRAPEUTIQUE

(30) Priority: 07.07.2014 US 201462021328 P
(43) Date of publication of application: 17.05.2017
(73) Proprietor: Targazyme Inc., Carlsbad, CA 92011 (US)
(72) Inventor: WOLFE, Stephen, D., Boyds, MD 20841 (US)
(74) Representative: Carpmaels & Ransford LLP
(86) International application number: PCT/US2015/039370
(87) International publication number: WO 2016/007506

(56) References cited:
- WO-A2-2005/017115
- US-A1- 2004 209 357
- US-A1- 2005 026 133
- US-A1- 2010 304 436
- US-A1- 2010 311 036
- US-A1- 2011 091 434
- US-A1- 2011 136 682
- "Fracture Mechanics: Twenty-Third Symposium", 1 January 1993, ASTM INTERNATIONAL, 100 Barr Harbor Drive, PO Box C700, West Conshohocken, PA 19428-2959, ISBN: 978-0-8031-1867-6, article K LEE ET AL: "Developing", pages: 133 - 133-35, XP055422083, DOI: 10.1520/STP24268S
- SHPALL E J ET AL: "Novel Cord Blood Transplant Therapies", BIOLOGY OF BLOOD AND MARROW TRANSPLANTATION, KLUGE CARDEN JENNINGS PUBLISHING, CHARLOTTESVILLE, VA, US, vol. 17, no. 1, 1 January 2011 (2011-01-01), pages S39 - S45, XP027580643, ISSN: 1083-8791, [retrieved on 20101015]

## Description

### BACKGROUND

Treating cells with an α1,3-fucosyltransferase and fucose donor increases their ability to bind to the class of adhesion proteins called selectins. During inflammation, ischemia or tissue damage, P-selectin and E-selectin cooperatively mediate leukocyte rolling and adhesion on vascular surfaces (reviewed in Zarbock et al. (2011) Blood, 118:6743-51). In most tissues, P-selectin and E-selectin are expressed on endothelial cells after stimulation of agonists, but they are expressed constitutively on bone marrow endothelial cells.

Selectins use α2,3-sialylated and α1,3-fucosylated glycans such as sialyl Lewis X (sLeX) on glycoproteins or glycolipids as ligands. For example, P-selectin binds to the N-terminal region of P-selectin glycoprotein ligand-1 (PSGL-1), which contains tyrosine sulfates and an O-glycan capped with sLex. E-selectin binds to one or more different sites on PSGL-1. To interact with E-selectin, PSGL-1 does not require tyrosine sulfation, but expression of sLex on O-glycans enhances binding. E-selectin also interacts with other ligands. An isoform of CD44 on HSCs has been shown to bind to E-selectin *in vitro* (Dimitroff et al. (2001) J Cell Biol., 153:1277-1286). Another potential ligand for E-selectin on HSCs is E-selectin ligand-1 (ESL-1) (Wild et al. (2001) J Biol Chem., 276:31602-31612). Each of these glycoprotein ligands is thought to carry sLeX structures.

Fucose is the terminal carbohydrate in sLeX and *ex vivo* fucosylation has been shown to increase the levels of cell surface sLeX as well as the ability of cells to extravasate from the vasculature into the surrounding tissues (Xia et al. (2004) Blood, 104:3091-6; Sackstein et al. (2008) Nat Med, 14:181-7; Sarkar et al. (2011) Blood, 118:e184-91; Robinson et al. (2012) Exp Hematol., 40:445-56; US Patent 7,332,334; US 2006/0210558; US Application 12/948,489).

The described methods of *ex vivo* fucosylation to date have involved treating cells just prior to intravenous injection into an animal or human. For example, currently there is a clinical trial being conducted ("ClinicalTrials.gov" Identifier NCT01471067) testing the utility of treating cord blood cells with α1,3-fucosyltransferase VI plus GDP-fucose prior to transplant in order to improve the ability of the cord blood cells to home and engraft into the bone marrow. In this application, cord blood is fucosylated at the point of care without expanding the cell population. The trial involves obtaining cord blood that is genetically matched to the recipient from a cord blood bank, thawing the cells and washing them free of cryoprotectants, treating with α1,3-fucosyltransferase VI plus GDP-fucose for 30 minutes at room temperature, washing the cells again, and infusing them into the patient through the intravenous route.

For many applications, however, it is advantageous to expand the number of cells prior to treatment. For example, the number of hematopoietic cells in cord blood is sufficient to engraft a child after transplantation but not an adult. For this reason, a number of attempts have been made to expand the number of engraftable cells by culturing the cord blood cells under various conditions prior to transplantation (reviewed in Dahlberg et al. (2011) Blood, 117:6083-90; and Delaney et al. (2013) Biol Blood Marrow Transplant, 19(1 Suppl):S74-8).

Despite intensive work, however, there is currently no method for expansion of hematopoietic cells that retains all the characteristics of the original cell population. Both the cell surface characteristics of the cells, as well as their *in vitro* and *in vivo* potencies, can change. For example, during *ex vivo* expansion, adhesion to N-cadherin, osteopontin and vascular cell-adhesion molecule-1, ligands present in bone marrow niches, is rapidly reduced, which may explain in part the reduced ability of expanded cells to engraft into the bone marrow (Kallinikou et al. (2012) Br J Haematol., 158:778-87). It is therefore clear that expanded hematopoietic cell populations are different than primary or unexpanded cell populations. To date, no studies have looked at whether the loss of adhesion molecules during *ex vivo* expansion affects the ability of the cells to be fucosylated or whether fucosylation can rescue the engraftment defects that occur with *ex vivo* expansion.

A similar situation exists with mesenchymal stromal cells (MSC). MSC represent a small percentage (0.001-0.01% of total nucleated cells) of bone marrow cells. However, current therapeutic doses of MSC require doses of 1-5 × 10⁶ MSCs/kg body weight and some applications may require even higher cell doses (>5 × 10⁶ MSCs/kg body weight) to be effective; it is therefore necessary to develop MSC expansion protocols that allow for the generation of up to 5-10 ×10⁸ MSCs from a limited starting volume of primary material.

However, *ex vivo* expansion of MSC can alter their therapeutic properties depending on the conditions used (Menard et al. (2013) Stem Cells Dev., 22:1789-801). In addition, a number of cell surface antigens (integrin α6, integrin αv, CD71,CD140b, CCR4, CD200, CD271, CD349 and CXCR7) are down-regulated with passage of MSC during passage under any of five GMP-compliant expansion conditions (Fekete et al. (2012) PLoS One, 7(8):e43255). To date, however, no studies have looked at whether the loss of these cell surface antigens during *ex vivo* expansion affects the ability of the cells to be fucosylated or whether fucosylation can improve homing and engraftment of MSC manufactured in large-scale expansion cultures.

Manufacture of cells for therapeutic use often involves expansion of a limited number of primary cells from either living or cadaveric donors in tissue culture. Tissues useful for obtaining such cells include, but are not limited to, cells isolated from bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth or cells derived from embryonic stem (ES) cells or induced pluripotent stem (iPS) cells.

A common method for isolation of cells derived from solid tissues is to treat the tissue with proteolytic enzymes such as collagenase that destroy the matrix holding the cells in the tissue and release them into the tissue culture medium; alternatively, mechanical methods such as sonication can be used.

Optionally, a population of cells may be selected by contacting the cells with one or more antibodies to cell surface antigens such as anti-CD34 or anti-STRO1 and separating the cells by methods known in the art such as fluorescent activated cell sorting (FACS) or magnetic bead isolation. Conveniently, the antibodies may be conjugated with markers, such as magnetic beads, that allow for direct separation; biotin, which can be removed with avidin or streptavidin bound to a support; fluorochromes, which can be used with a fluorescence activated cell sorter (FACS), or the like, to allow for ease of separation of the particular cell type. Any technique may be employed that is not unduly detrimental to the viability of the remaining cells. Rather than using antibodies that bind to the desired cell population, it is possible to negatively select by using antibodies that bind to the undesired cell populations.

The resulting cells are then either grown in suspension cultures (the typically desired method for cells such as hematopoietic, immune or lymphoid cells) or as attached cells (the typically desired method for cells that attach to tissue culture plastic such as MSCs, adipose stem cells, neuronal stem cells). Attached cells may be grown in flasks, roller bottles, cell factories, or on microcarrier beads that are then kept in suspension in disposable bags, stirred suspension bioreactors or wave bioreactors. Other methods known in the art include, but are not limited to, growing cells in hollow fiber devices, in bioreactors that can be rigid-walled stirred-tanks, rotating wall, parallel plates, or fixed and fluidized bed reactors; or in automatic cell processing units such as the Aastrom REPLICELL^{®} System (Aastrom Biosciences, Ann Arbor, MI) (see Rodrigues et al. (2011) Biotechnol Adv., 29:815-29 for review of these different methodologies).

The nature of the cells produced during manufacture can differ widely depending on the conditions used. For MSC expansion, fetal bovine serum (FBS) is often included in the culture medium. As a product obtained after the clotting of whole blood and release of platelet and other blood cell products, serum is a pathological fluid not normally seen in the body except for wound conditions. As a result, MSCs or other cells manufactured in the presence of serum see biologically active factors (e.g., platelet-derived cytokines and other products) that they would not normally see in situ under normal homeostatic conditions. This is also true for cells grown in human platelet lysate, which can be used as a substitute for FBS when cells are produced under cGMP conditions. Cells grown in the presence of serum or platelet lysate therefore have properties that are different from primary cells obtained from tissues.

Attempts have been made to develop serum-free media to grow MSC and other cell types but these present a different set of problems. Cells normally exist *in vivo* in a complex environment in which they constantly receive signals from their environment. They may exist attached to extracellular matrix, be in close contact with other cell types, and be bathed in a complex proteinaceous fluid particularly to the organ, blood or lymph in which they are located. In comparison, existing serum-free media have few proteins and do not recapitulate the in situ environment. Moreover, the substrate for attached cells - usually tissue culture plastic, glass and the like - provide a very different environment than cells normally experience in situ. In many cases, cells flatten out to maximize adherence to the tissue culture substrate and as a result lose the cuboidal structure they normally have *in vivo* that is important to maintain function.

Regardless of the manufacturing process, therapeutic cells need to satisfy strict regulatory guidelines. Since expansion of cells is considered to be more than minimal manipulation, cells that are expanded are more strictly regulated than those that are simply obtained from a donor and given to a recipient with only minimal manipulation. In the U.S., therapeutic cells must be manufactured in a manner consistent with Current Good Manufacturing Practice (cGMP) regulations enforced by the US Food and Drug Administration (FDA). Cells that have been expanded are considered in the context of human cells, tissues, or cellular and tissue-based products (HCT/Ps). Therefore, cell production must be in compliance with The Code of Federal Regulation (CFR), Title 21, Part 1271 and in accordance with current Good Tissue Practice (cGTP) requirements as described in 'Current Good Tissue Practice (CGTP) and Additional Requirements for Manufacturers of Human Cells, Tissues, and Cellular and Tissue-Based Products (HCT/Ps). In Europe, expanded cells are considered as advanced therapy medicinal products (ATMPs), as defined by the European Regulation EC 1394/2007. Depending on the source, manufacturing process and intended application, expanded cells may be considered somatic-cell therapy products or tissue-engineered products. The European Regulation EC 1394/2007 refers to the European cGMP guidelines and is in compliance with the 2003/94/EC directive on medicinal products for human use as well as directive 2002/98/EC setting standards of quality and safety for the collection, testing, processing, storage and distribution of human blood and blood components.

The nature of cells grown under cGMP-compliant conditions can differ substantially from cells grown under laboratory conditions. Under laboratory conditions, cells are usually grown in 5 - 10% carbon dioxide (CO₂) in tissue culture medium containing 5 - 10% fetal bovine serum and levels of glucose higher than those usually found in non-diabetic individuals *in vivo.* The medium used under laboratory conditions is usually one of the standard laboratory media such as Roswell Park Memorial Institute (RPMI) 1640, Dulbecco's modified Eagle's medium (DMEM) and the like; cells are adapted to grow in one of these standard media. Under laboratory conditions, the cells are grown for a period of time until they begin to exhaust the nutrients in the tissue culture medium which are then replaced either by replacing 50%-95% of the medium.

The high oxygen tension used in laboratory conditions can cause oxidative stress to cells. Nutrient and metabolite concentrations, which can fluctuate widely under laboratory conditions, can also influence cell behavior.

In contrast, cGMP process development optimizes each of these parameters, as well as many others, for each cell type (see Rodrigues et al. for review). The culture vessels used for cGMP manufacture are often very different than used under laboratory conditions and often involve bioreactors as opposed to tissue culture flasks. The tissue culture medium components are usually optimized for each cell type rather than using one off-the-shelf tissue culture media, and growth factors and other additives are used that are themselves produced under cGMP conditions. Manufacturers that are produced under cGMP conditions generally strive to eliminate xenogeneic additives such as FBS that are commonly used under laboratory conditions. Feeding parameters, growth factors, and oxygenation are optimized for each cell type during cGMP process development, and fluctuations in nutrient and metabolite concentrations are kept within tight limits. Finally, the scale of expansion for cGMP processes are often orders of magnitude larger than occurs under normal laboratory conditions.

For these reasons, manufacture of therapeutic cells is not simply a matter of scaling up laboratory-based methods. Instead, detailed optimization studies must be conducted at every step of process development, and observations made under academic laboratory conditions may not necessarily apply to cells grown under cGMP-compliant conditions. Further, as indicated above (e.g., Kallinikou et al., Menard et al., and Fekete et al.), the nature of cells may change with large-scale expansion even under cGMP-compliant conditions, which are usually optimized for cell growth and not for function. Therefore results obtained with primary cells or with cells grown under laboratory conditions may not apply to cells expanded to the extent and under the conditions used in large-scale cGMP manufacturing processes.

To date, the optimal methods for fucosylation of expanded cell populations have not been determined. In particular, optimal methods for fucoslation have not been determined for cells grown under cGMP conditions. Depending on the intended use, the fucosylation step can be incorporated into different points during the manufacture of the therapeutic cells. For some applications, it is advantageous to manufacture cells and deliver them directly to the patient without cryopreservation. Examples of such applications include, but are not limited to, *ex vivo* expansion of hematopoietic stem cells or immune cells, mesenchymal stem cells, adipose-derived stem cells, dental pulp-derived stem cells, muscle cells, amniotic cells, endometrial cells, neural stem cells and cells derived from induced pluripotent stem (iPS) cells, particularly when the cells being given to the patient are autologous (i.e., where the cells are derived from the patient or a genetically identical individual).

In some cases it is advantageous to manufacture the cells at a central processing center. This method involves growing a large batch of cells *in vitro,* fucosylating them under controlled conditions and freezing aliquots for distribution to the clinical center where they will be administered. Examples of such applications include, but are not limited to, mesenchymal stromal cells (MSC), adipose-derived stem cells, dental pulp-derived stem cells, muscle cells, amniotic cells, endometrial cells and neural stem cells and cells derived from embryonic stem (ES) cells or induced pluripotent stem (iPS) cells, particularly when the cells being given to the patient are allogeneic (i.e., from a donor who is genetically different from the recipient). In these cases there are economic, quality control, and distribution advantages to being able to grow a large batch of cells, fucosylate them in bulk, and cryopreserve them in aliquots prior to distribution to medical centers for administration to patients. Cryopreservation of cells involves adding cryoprotectants to the medium and using a controlled rate of freezing, then storing the cells at low temperatures, usually in liquid nitrogen freezers. Cryoprotectants are substances used to protect biological tissue from freezing damage caused by the formation of ice crystals. Cryoprotectants fall into two general categories: permeating cryoprotectants, which can pass through cell membranes, and non-permeating cryoprotectants, which do not penetrate the cell membrane and act by reducing the hyperosmotic effect present in the freezing procedure. Examples of permeating cryoprotectants include, but are not limited to, dimethyl sulfoxide (Me₂SO or DMSO), glycerol, sucrose, ethylene glycol, 1,2-propanediol, and any combinations thereof. Examples of non-permeating cryoprotectants include, but are not limited to, hydroxyethyl starch, albumin, sucrose, trehalose, dextrose, polyvinyl pyrrolidone, and any combinations thereof.

The most widely used permeative cryoprotectant is DMSO, which is a hygroscopic polar compound that prevents the formation of ice crystals during freezing. DMSO is often used in combination with a non-permeative agent such as autologous plasma, serum albumin, and/or hydroxyethyl starch. By using a mixtures of different cryoprotectants the toxicity of the solution is decreased, hence rendering the solution more effective than single-agent cryoprotectants. For example, the cryopreservation method that is most commonly employed for cells includes a freezing medium consisting of 5 - 20% DMSO in the presence of either animal or human serum. The use of a controlled-rate freezing technique at 1 to 2 °C/minute and rapid thawing is considered standard. This can involve the use of a controlled rate freezer that reduces temperature at that rate or a passive cooling device such as a mechanical refrigerator, generally at -80 °C, to cool the cells (so-called dump-freezing) to generate cooling rates similar to those adopted in controlled rate freezing.

Rubinstein and colleagues at the New York Blood Center developed an optimized protocol for using DMSO to freeze cord blood units (Rubinstein et al. (1995) PNAS, 92:10119-22). Hetastarch was added to the unit followed by centrifugation to remove excess red blood cells and plasma and achieve a uniform final volume of 20 mL containing essentially all the stem and progenitor cells (US Patent No. 5,789,147). After volume reduction of the cord blood unit, 5 mL of cryopreservation solution (0.85 NaCl, 50% DMSO [Cryoserv; Research Industries, Salt Lake City, UT] and 5% Dextran 40 [Baxter Healthcare, Deerfield, IL]) was added to the cell suspension slowly and with continuous mixing. Units were frozen using a controlled-freeze stored in cryogenic tanks within the liquid phase of liquid nitrogen. Similar methods are in use for a wide variety of cell types (reviewed in Hunt (2011) Transfus Med Hemother, 38:107-123).

Despite such detailed studies of cryopreservation methods to maintain cell viability, however, there have been no studies that have investigated the retention of cell surface fucosylation after cryopreservation.

The exact cell surface components that are fucosylated after *ex vivo* treatment with an α1,3-fucosyltransferase and fucose donor have not been fully characterized for any cell type. It is known for some cells that they involve both glycolipids and glycoproteins, and some of the major targets of fucosylation, such as PSGL-1, CD44 and ESL-1, have been identified, as described above. However, the full spectrum of proteins and glycolipids that is fucosylated after *ex vivo* treatment has not been well defined for any cell type.

Faint et al. (J Immunother. (2011) 34:588-96) disclosed that cryopreservation of lymphocytes affects cell surface antigens. These authors observed reduced levels of CD69, a transmembrane protein that plays a critical role in lymphocyte egress from tissues, and the chemokine receptor CXCR4, a major chemoattractant receptor, increased after thawing, whereas levels of CD62L, an adhesion protein, and CXCR3, another chemoattractant protein, were reduced. These changes were associated with modulation of the ability of lymphocytes to migrate across cytokine-stimulated monolayers of endothelium toward recombinant CXCL11 and CXCL12. Thus cryopreservation and thawing of lymphocytes induces changes in their adhesive phenotype and modulated their ability to migrate across endothelial monolayers.

Similarly, Koenigsmann et al. (Bone Marrow Transplantation (1998) 22:1077-1085) studied adhesion molecules on CD34+ cells before and after cryopreservation and found that freezing markedly reduced the fraction of CD34+ cells with L-selectin (CD62L) expression from 62 to 11% and also diminished the fluorescence intensity for the integrin subunits CD29 and CD49d. Decreases in L-selectin were also observed by Hattori et al. (Exp. Hemat. 29 (2001) 114-122).

Campbell et al. (Clin Vaccine Immunol. (2009) 16:1648-53) found that cryopreservation significantly reduced the expression of both PD-1 and PD-L1 on PBMC-derived CD3+/CD8+ T cells and CD45+/CD14+ monocytes.

Aoyagi et al. (J Craniofac Surg. (2010) 21:666-78) found significant changes in expression of the cell surface protein CD271 in MSCs after cryopreservation. DMSO can rapidly induce neuronal-like morphology in MSCs and increased expression of neuronal markers such as GFAP, nestin, neuronal nuclear antigen (NeuN) and neuron-specific enolase (NSE), (Mareschi et al. (2006) Exp Hematol., 34(11):1563-72; and Neuhuber et al. (2004) J Neurosci Res., 77:192-204).
US 2011/091434 describes "methods, compositions of matter, and kits useful for augmentation of homing and engraftment of stem, progenitor and mature cells through modification of cellular membrane properties following ex vivo treatment", that "may be used for the treatment of a wide variety of disorders in which augmentation of cell trafficking, homing and engraftment is desired".
US 2011/136682 describes "methods of evaluating CHO cells and producing recombinant glycoproteins".

While all these studies disclosed that cryopreservation can alter adhesive properties and cell surface antigen expression, none looked at whether it affected the levels of cell surface fucosylation. Since cryopreservation can alter cell surface adhesion and other molecules on a variety of cell types in manners not predictable *a priori,* and since the nature of the cell surface components that become fucosylated after treatment with α1,3-fucosyltransferase and fucose donor have not been fully defined, the effects of cryopreservation on cell surface fucosylation can only be determined empirically. To date, no studies have been published in either the scientific or patent literature that address this question.

The extent to which different cell types can be fucosylated after *ex vivo* expansion, and the extent to which cell surface fucosylation is stable to cryopreservation, can only be empirically determined for each cell type. As indicated by the previous discussion, *ex vivo* expansion and cryopreservation each can affect the expression and function of a number cellular adhesion molecules and other cell surface components; these changes are cell-type specific and cannot be predicted *a priori.* Whereas proteins like L-selectin might recover from the loss due to cryopreservation and thawing after short term incubation (Hattori et al.) this is unlikely to happen with fucosylation levels after *ex vivo* fucosylation since there are no internal stores of fucosylated proteins to replace the ones that were exposed to the exogenous enzyme and fucose donor. The identification of conditions for manufacture and cryopreservation of cells with increased fucosylation levels is the subject of the present application.

### BRIEF DESCRIPTION OF THE DRAWINGS

Figure 1 graphically illustrates a comparative analysis of the kinetics of cell surface fucosylation by FTVI or FTVII of mononuclear cells from thawed human cord blood.
Figure 2 graphically illustrates a comparative analysis of the kinetics of cell surface fucosylation by FTVI or FTVII of human mesenchymal stem cells (MSCs).
Figure 3 graphically illustrates a comparative analysis of the kinetics of cell surface fucosylation of purified cord blood-derived CD34+ cells by FTVI or FTVII.
Figure 4 graphically illustrates a comparative analysis of the kinetics of cell surface fucosylation by FTVI or FTVII of fresh cultured neural stem cells (NSCs).
Figures 5 and 6 graphically illustrate a comparative analysis of the kinetics of cell surface fucosylation of human thawed cord blood-derived mononuclear cells by FTVI (Figure 5) or FTVII (Figure 6).
Figure 7 graphically illustrates an analysis of the effects of FTVI treatment versus sham treatment on fucosylation of human endothelial progenitor cells (EPCs).
Figure 8 graphically illustrates an analysis of the effects of FTVI treatment on fucosylation of human amniotic stem cells.
Figure 9 illustrates an analysis of the effects of FTVI treatment on fucosylation of human adipose-derived stem cells.
Figure 10 graphically illustrates an analysis of the effects of fucosylation of human MSCs either before or after trypsinization.
Figure 11 illustrates the effect of incubating hNK cells with varying concentrations of FTVI on the Level (%) of Fucosylation. hNK cells were expanded for 14 days, harvested, washed, and incubated with varying concentrations of FTVI ranging from 5 µg/mL to 25 µg/mL. With the addition of GDP-fucose (final concentration of 1 mM in all samples), cells were incubated for 30 minutes at room temperature, followed by analysis of the extent of fucosylation with CLA-FITC stain in addition to analyzing other cell surface markers (CD62L, CD44, CD16, CD56 and PSGL) characteristic of NK cells.
Figure 12 illustrates the effect of incubating control and TZ101-treated human NK cells on fluid phase binding to E-selectin chimera. Expanded hNK cells were incubated without or with 5, 10, 25, and 50 µg/mL TZ101 at 2.5 x 10⁶ NK cells/mL for 30 minutes at room temperature, washed, and resuspended. 1 µg/10⁵ NK cells was then incubated with human or mouse E-selectin/Fc chimeric protein for 30 minutes at 4°C and stained with CLA, CD44, human IgG, and Annexin V.
Figure 13 illustrates an examination of the stability of fucosylated NK Cells at 48 hours following treatment with TZ101. hNK cells were expanded for 18 days, harvested, washed, and incubated with FTVI at 25 µg/mL. Following the addition of GDP-fucose (final concentration of 1 mM), cells were incubated for 30 minutes at room temperature, followed by analysis of the extent of fucosylation with CLA-FITC stain at 1 hour and 48 hours after being maintained in culture media.
Figure 14 illustrates a comparative analysis of cytotoxic potential of control versus fucosylated NK cells. hNK cells were expanded for 14 days, harvested, washed, and incubated with IL-2 for 24 hours prior to incubation with indicated cell lines. Toxicity was measured following the incubation of K562 cells and MM1S cells with either control or TZ101-fucosylated hNK cells. Cytotoxicity was monitored at the end of 4 hours of incubation with the measurement of chromium release.

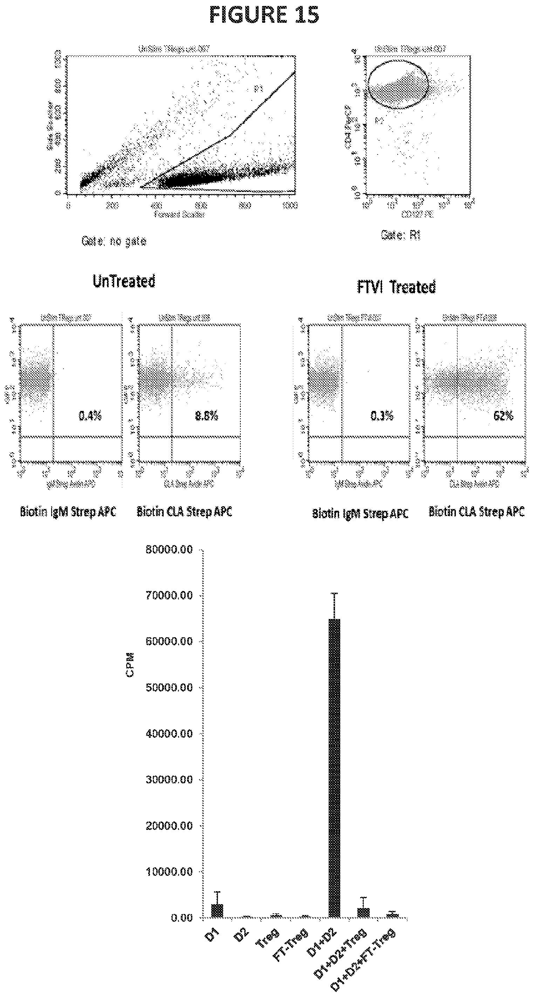

Figure 15A illustrates the fucosylation of Regulatory T (T_{reg}) cells. The left side of each dot plot shows the isotype control, while the right side shows staining along with the expression of the percent CLA positive cells. Treatment with TZ101 (FTVI + GDP-fucose) increased the expression of cell surface sLeX units from 8.8% to 62%, as detected with HECA-452 anti-CLA antibody stain. Figure 15B illustrates that fucosylated (FT) T_{reg} cells maintain their suppressive function. PBMCs from two donors were cultured together to generate MLR (D1+D2)_{.} Addition of T_{reg} cells or FT-T_{reg} cells to the donor mixture (D1+D2) at a ratio of 1:1 significantly suppressed MLR. Y-axis denotes counts per minute (CPM) (Mean±SEM, n=3).
Figure 16 illustrates expansion of cytotoxic T cells against CG1 (CG1-CTL) and fucosylation thereof. Fucosylation levels were measured using flow cytometry and anti-CLA FITC. Non-treated cells exhibited 4% fucosylation, whereas cells treated with TZ101 exhibited 100% fucosylation.

### DETAILED DESCRIPTION

The present invention is defined by the appended claims. Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (2nd ed., Cold Spring Harbor Laboratory Press, Cold Spring Harbor, N.Y. (1989) and Coligan et al. Current Protocols in Immunology (Current Protocols, Wiley Interscience (1994)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

All patents, published patent applications, and non-patent publications mentioned in the specification are indicative of the level of skill of those skilled in the art to which this invention pertains.

All of the compositions and/or methods disclosed and/or claimed herein can be made and executed without undue experimentation in light of the present disclosure. While the compositions and methods of the inventive concept(s) have been described in terms of particular, non-limiting embodiments, it will be apparent to those of skill in the art that variations may be applied to the compositions and/or methods and in the steps or in the sequence of steps of the method described herein without departing from the invention.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The singular forms "a," "an," and "the" include plural referents unless the context clearly indicates otherwise. Thus, for example, reference to "a compound" may refer to 1 or more, 2 or more, 3 or more, 4 or more or greater numbers of compounds. The term "plurality" refers to "two or more." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. For example but not by way of limitation, when the term "about" is utilized, the designated value may vary by ± 20% or ± 10%, or ± 5%, or ± 1%, or ± 0.1% from the specified value, as such variations are appropriate to perform the disclosed methods and as understood by persons having ordinary skill in the art. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z. The use of ordinal number terminology (i.e., "first", "second", "third", "fourth", etc.) is solely for the purpose of differentiating between two or more items and is not meant to imply any sequence or order or importance to one item over another or any order of addition, for example.

As used in this specification and claim(s), the terms "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, the term "substantially" means that the subsequently described event or circumstance occurs at least 90% of the time, or at least 95% of the time, or at least 98% of the time.

As used herein, "Current Good Manufacturing Practice" or "cGMP" refers to the Current Good Manufacturing Practice regulations enforced by the US Food and Drug Administration (FDA) or equivalent regulatory authorities in non-US countries. cGMP regulations provide for systems that assure proper design, monitoring, and control of manufacturing processes and facilities. Adherence to the cGMP regulations assures the identity, strength, quality, and purity of drug products by requiring that manufacturers of medications adequately control manufacturing operations. This includes establishing strong quality management systems, obtaining appropriate quality raw materials, establishing robust operating procedures, detecting and investigating product quality deviations, and maintaining reliable testing laboratories.

As used herein, the term *"ex vivo* expansion" or "expansion" refers to a method of growing a cell population in tissue culture that increases the number of cells in that population. Cells that have undergone *ex vivo* expansion are referred to as "expanded".

As used herein, the term "fucosylation" refers to the treatment of a population of cells with an α1,3-fucosyltransferase and fucose donor under conditions that increase the ability of the cells to bind to a selectin or that increase the reactivity of the cells with an antibody known in the art to bind to sLeX including, but not limited to, the HECA-452 monoclonal antibody. Cells that have been treated with an α1,3-fucosyltransferase and fucose donor and then exhibit increased binding to selectins or to the HECA-452 monoclonal antibody or to another antibody specific for sLeX are referred to as being "fucosylated". As used herein, "fucosylation" can also refer to the levels of sLeX present on a cell population.

As used herein, the term "hematopoeitic stem and progenitor cells" or "HSPC" refers to a cell population derived from bone marrow, cord blood or mobilized peripheral blood that is used to reconstitute the hematopoietic system of a patient. As used herein, the term hematopoeitic stem and progenitor cells" or "HSPC" includes carlecortemcel-L.

As used herein, the term "mesenchymal stromal cell" or "MSC" refers to cells that meet the definition set in 2006 by The International Society for Cellular Therapy (ISCT): (1) adherence to plastic, (2) expression of CD73, CD90, and CD105 antigens, while being CD14, CD34, CD45, and HLA-DR negative, and (3) ability to differentiate to osteogenic, chondrogenic and adipogenic lineage (Dominici et al. (2006) Cytotherapy, 8:315-317). As used herein, "mesenchymal stromal cell" or "MSC" is synonymous with "mesenchymal stem cell," and thus said terms are used interchangeably herein. As used herein, "MSC" can be used as either singular or plural. As used herein, "mesenchymal stromal cell" or "MSC" can be derived from any tissue including, but not limited to, bone marrow, adipose tissue, amniotic fluid, endometrium, trophoblast-derived tissues, cord blood, Wharton jelly, and placenta. As used herein, "mesenchymal stromal cell" or "MSC" includes cells that are CD34 positive upon initial isolation from tissue but satisfy the ISCT criteria after expansion. As used herein, "MSC" includes cells that are isolated from tissues using cell surface markers selected from the list comprised of NGF-R, PDGF-R, EGF-R, IGF-R, CD29, CD49a, CD56, CD63, CD73, CD105, CD106, CD140b, CD146, CD271, MSCA-1, SSEA4, STRO-1 and STRO-3 or any combination thereof, and satisfy the ISCT criteria either before or after expansion. As used herein, "mesenchymal stromal cell" or "MSC" includes cells described in the literature as bone marrow stromal stem cells (BMSSC), marrow-isolated adult multipotent inducible cells (MIAMI) cells, multipotent adult progenitor cells (MAPC), mesenchymal adult stem cells (MASCS), MULTISTEM^{®} (Athersys, Inc., Cleveland, OH), PROCHYMAL^{®} (Osiris Therapeutics, Inc., Columbia, MD), remestemcel-L, Mesenchymal Precursor Cells (MPCs), Dental Pulp Stem Cells (DPSCs), PLX cells, PLX-PAD, ALLOSTEM^{®} (Allosource, Centennial, CO), ASTROSTEM^{®} (Osiris Therapeutics, Inc., Columbia, MD), Ixmyelocel-T, MSC-NTF, NurOwn^{™} (Brainstorm Cell Therapeutics Inc., Hackensack, NJ), STEMEDYNE^{™}-MSC (Stemedica Cell Technologies Inc., San Diego, CA), STEMPEUCEL^{®} (Stempeudics Research, Bangalore, India), StempeuceICLI, StempeucelOA, HiQCell, Hearticellgram-AMI, REVASCOR^{®} (Mesoblast, Inc., Melbourne, Australia) CARDIOREL^{®} (Reliance Life Sciences, Navi Mumbai, India), CARTISTEM^{®} (Medipost, Rockville, MD), PNEUMOSTEM^{®} (Medipost, Rockville, MD), PROMOSTEM^{®} (Medipost, Rockville, MD), Homeo-GH, AC607, PDA001, SB623, CX601, AC607, Endometrial Regenerative Cells (ERC), adipose-derived stem and regenerative cells (ADRCs) obtained with the CELUTION^{®} System (Cytori Therapeutics, Inc., San Diego, CA), perivascular-derived cells, and pericyte-derived cells. As used herein, "mesenchymal stromal cell" or "MSC" includes cells that only satisfy one or more of the ISCT criteria when cultured under one set of conditions but satisfy the full set of ISCT criteria when cultured on plastic tissue culture flasks in the presence of tissue culture medium containing 10% fetal bovine serum.

As used herein, the term "muscle stem cells" refers to a cell population derived from muscle, including striated muscle, smooth muscle, cardiac muscle, muscle satellite cells or bone marrow cells reprogrammed to form muscle. As used herein, the term "muscle stem cells" includes MyoCell^{®} (Bioheart, Inc., Sunrise, FL), MyoCell^{®} SDF-1, C3BS-CQR-1, and CAP-1002.

As used herein, "natural killer cells" or "NK" cells refers to a cell population that lacks CD3 and expresses CD56 and/or NKp46.

As used herein, "neural stem cells" or "NSC" refers to a cell population capable of differentiating into neural cells or glial cells. As used herein, the term "neural stem cells" includes Q-Cells^{®} (Q Therapeutics Inc., Salt Lake City, UT), NSI-566, HuCNS-SC^{®} (Stem Cells, Inc., Newark, CA), and ReN001.

As used herein, "patient" is used broadly to refer to any animal in need of therapeutic cells to ameliorate a condition, disease or injury. The animal can be a mammal, a bird, a fish, a reptile or any other animal. Some non-limiting examples of mammals include humans and other primates, equines such as horses, bovines such as cows, ovines such as sheep, caprines such as goats, canines such as dogs, felines such as cats, rodents such as mice or rats, and other mammals such as rabbits, Guinea pigs, and the like.

As used herein, "physiologically balanced salt solution" refers to a solution or medium where the concentrations of salts and other components are adjusted such that the solution or medium is isotonic with human cells, with osmolarity approximately 280 to 310 mOsmol/L, and is at a physiological pH, approximately pH 7.3 - 7.4. Examples of physiologically balanced salt solutions include, but are not limited to, Hank's basic salt solution, Alpha Minimum Essential Medium (αMEM), Dulbecco's Minimum Essential Medium (DMEM), Iscove's Modified Dulbecco's Medium (IMDM) and PlasmaLyte solutions such as PlasmaLyte A.

As used herein, "therapeutic cells" refers to an expanded cell population that ameliorates a condition, disease, and/or injury in a patient. Therapeutic cells may be autologous (i.e., derived from the patient), allogeneic (i.e., derived from an individual of the same species that is different than the patient) or xenogeneic (i.e., derived from a different species than the patient). Therapeutic cells may be homogenous (i.e., consisting of a single cell type) or heterogenous (i.e., consisting of multiple cell types). The term "therapeutic cell" includes both therapeutically active cells as well as progenitor cells capable of differentiating into a therapeutically active cell.

The present invention relates to a method of cryopreserving fucosylated therapeutic cells, the method comprising the steps of: isolating therapeutic cells; fucosylating the therapeutic cells by contacting them with an effective amount of an α1,3-fucosyltransferase and GDP-fucose; mixing the fucosylated therapeutic cells with a therapeutic cell cryopreservation composition comprising a physiologically balanced salt solution, at least one permeating cryoprotectant, and at least one non-permeating cryoprotectant to form a mixture; and freezing the mixture by cooling at a rate in a range of from 1°C to 2°C per minute from 37°C to -80°C to produce a frozen cell suspension, and transferring the frozen cell suspension to storage in the presence of liquid nitrogen. Also disclosed generally are compositions for and methods of manufacturing therapeutic cells that are treated with an α1,3-fucosyltransferase and fucose donor and exhibit enhanced migration and engraftment when administered *in vivo* compared to their non-fucosylated counterparts.

Embodiments also relate to the commercial provision of the possibility to manufacture and optionally to cryopreserve the therapeutic cells under Current Good Manufacturing Practice (cGMP) regulations enforced by the United States (US) Food and Drug Administration (FDA) or the equivalent regulatory authority in non-US countries. The therapeutic cells are useful for treating a variety of diseases and disorders including, but not limited to, ischemic conditions (e.g., limb ischemia, congestive heart failure, cardiac ischemia, kidney ischemia and ESRD, stroke, and ischemia of the eye), conditions requiring organ or tissue regeneration (e.g., regeneration of liver, pancreas, lung, salivary gland, blood vessel, bone, skin, cartilage, tendon, ligament, brain, hair, kidney, muscle, cardiac muscle, nerve, and limb), inflammatory diseases (e.g., heart disease, diabetes, spinal cord injury, rheumatoid arthritis, osteo-arthritis, inflammation due to hip replacement or revision, Crohn's disease, and graft versus host disease) autoimmune diseases (e.g., type 1 diabetes, psoriasis, systemic lupus, and multiple sclerosis), a degenerative disease, a congenital disease hematologic disorders such as anemia, neutropenia, thrombocytosis, myeloproliferative disorders or hematologic neoplasms and cancer such as leukemia and lymphoma.

The therapeutic cells may be isolated from bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth, cells derived from embryonic stem (ES) cells or induced pluripotent stem (iPS) cells, or any combination thereof.

In a particular, non-limiting embodiment, the isolated therapeutic cells are differentiated embryonic stem cells and/or differentiated induced pluripotent stem cells.

In particular, one embodiment relates to methods of mass producing such cells, treating them with an effective amount of an α1,3-fucosyltransferase and the fucose donor GDP-fucose (e.g. α1,3-fucosyltransferase VI or α1,3-fucosyltransferase VII together with the fucose donor GDP-fucose), and then cryopreserving them under conditions where the enhanced levels of cell surface fucosylation resulting from the enzyme treatment are retained after thawing the cells.

The present invention can also be used for veterinary purposes since there is a parallelism between the mechanisms involved in enhanced binding to selectins after fucosylation of selectin ligands between humans and animals.

In the methods contemplated herein, the fucosyltransferase may be selected from the group comprised of an α1,3-fucosyltransferase III, an α1,3-fucosyltransferase IV, an α1,3-fucosyltransferase V, an α1,3-fucosyltransferase VI, an α1,3-fucosyltransferase VII, an α1,3-fucosyltransferase IX, an α1,3-fucosyltransferase X, and an α1,3-fucosyltransferase XI, or any combination thereof. The fucose donor is GDP-fucose.

In one embodiment the method comprises the steps of isolating therapeutic cells, expanding the therapeutic cells, and fucosylating the quantity or population of therapeutic cells by contacting them *in vitro* with an effective amount of an α1,3-fucosyltransferase and the fucose donor GDP-fucose. The fucosylated therapeutic cells have enhanced binding to P-selectin or E-selectin. The fucosylated therapeutic cells are cryopreserved under conditions that retain the enhanced binding to P-selectin or E-selectin after thawing the cells.

The present invention is a method of cryopreserving fucosylated therapeutic cells. In the method, therapeutic cells are isolated and fucosylated by contacting them with an effective amount of an α1,3-fucosyltransferase and the fucose donor GDP-fucose. The fucosylated therapeutic cells are then frozen in a therapeutic cell cryopreservation composition comprising a physiologically balanced salt solution and a cryoprotectant.

The method may further include the step of expanding the therapeutic cells prior to fucosylation. When the cells are expanded, in a particular, non-limiting embodiment, the physiologically balanced salt solution in which the cells are frozen may be the tissue culture medium in which the cells are expanded. In addition, the physiologically balanced salt solution may further contain protein. Non-limiting examples of proteins that may be utilized include fetal bovine serum, horse serum, human serum, human platelet lysate, bovine albumin, human albumin, and any combinations thereof.

In a particular, non-limiting embodiment, the freezing step includes cooling the therapeutic cells in the cell cryopreservation composition at a rate of about 1°C per minute from about 37°C to about -80°C to produce a frozen cell suspension, and then transferring the frozen cell suspension to storage in the presence of liquid nitrogen. In addition, the therapeutic cells may be frozen using a vitrification method.

In a particular, non-limiting embodiment, adherent cells are first removed from the tissue culture plastic or microbead or other substrate on which they are grown, treated with an α1,3-fucosyltransferase and the fucose donor GDP-fucose and then cryopreserved. It is a surprising finding that removal of cells from tissue culture plastic and other substrates by exposing them to trypsin followed by fucosylation is a more effective method than fucosylation of cells while attached to tissue culture plastic and then removing them with trypsin.

In a particular, non-limiting embodiment, the methods are performed under cGMP conditions.

In a particular, non-limiting embodiment, the therapeutic cells are cells isolated from bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth, cells derived from embryonic stem (ES) cells or induced pluripotent stem (iPS) cells, or any combination thereof.

In a particular, non-limiting embodiment, the therapeutic cells are selected from hematopoietic stem cells, immune cells, mesenchymal stem cells, muscle cells, amniotic cells, endometrial cells, neural stem cells, natural killer (NK) cells, T cells, B cells, or any combination thereof. For example, but not by way of limitation, the therapeutic cells may be T cells (including but not limited to, regulatory T cells and cytotoxic T cells (for example, but not by way of limitation, CD8+ cytotoxic T cells)), NK cells, B cells, CD38+ cells, neural stem cells, or any combination thereof, wherein said cells are fucosylated by fucosyltransferase VII (FT VII). It is a surprising finding that some cells are preferentially fucosylated with FT VII instead of FT VI. This is unexpected given the *in vitro* fucose donor specificities of the enzymes - whereas FucT-VI is active on both neutral and 3'-sialylated fucose donors, FucT-VII acts on only the 3'-sialylated type 2 chain. *A priori,* one would therefore expect that FTVI would fucosylate cells to approximately the same extent as FTVII; this was observed for some cells but not for others.

In one embodiment hematopoietic cells that have been expanded are mixed with unexpanded fucosylated hematopoietic cells. It is a surprising finding that a mixture of fucosylated and non-fucosylated expanded hematopoietic cells is more effective than either population used alone.

In one embodiment natural killer cells are expanded and then fucosylated. Until the filing of the present application there has been neither a description nor suggestion that natural killer cells can be fucosylated *ex vivo.*

Until the present disclosure, there has been neither a description nor suggestion towards the development of a cryopreservation method for fucosylated therapeutic cells. Furthermore, the inventors surprisingly found that by following the cryoprotection method of the present invention, therapeutic cells with a high retention of fucosylation are recovered after cryopreservation.

In one embodiment the method comprises the steps of isolating a quantity or population of therapeutic cells, expanding the therapeutic cells in tissue culture, treating the quantity or population of therapeutic cells *in vitro* with an α1,3-fucosyltransferase and the fucose donor GDP-fucose, wherein the treated therapeutic cells have enhanced binding to P-selectin and E-selectin, and then cryopreserving the cells. Furthermore, the therapeutic cells are typically characterized as comprising P-selectin glycoprotein ligand-1 (PSGL-1), CD44, and/or other selectin ligands that do not effectively bind to P-selectin or E-selectin. The therapeutic cells, in their untreated state prior to fucosylation as described herein, have reduced retention in inflamed, ischemic, or damaged tissues.

In a particular, non-limiting embodiment, the therapeutic cells are derived from the list comprising bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue and exfoliated teeth, though they may be derived from cells grown in tissue culture or are cells derived from embryonic stem (ES) cells or induced pluripotent stem (iPS) cells. The therapeutic cells may also be any combination of the above.

In a particular, non-limiting embodiment, the therapeutic cells are expanded under cGMP conditions.

As noted above, after the fucosylation treatment described herein, the treated therapeutic cells have enhanced binding to P-selectin or E-selectin, as compared to untreated therapeutic cells. Enhanced binding to P-selectin (or E-selectin) is defined as at least 10% of the treated therapeutic cells having fluorescence in a P-selectin (or E-selectin, respectively) binding assay which is greater than a predetermined fluorescence threshold (as defined below). In another embodiment, at least 25% of the treated therapeutic cells exceed the predetermined fluorescence threshold. In another embodiment, at least 50% of the treated therapeutic cells exceed the predetermined fluorescence threshold. In another embodiment, at least 75% of the treated therapeutic cells exceed the predetermined fluorescence threshold. In another embodiment, at least 90% of the treated therapeutic cells exceed the predetermined fluorescence threshold. In another embodiment, at least 95% of the treated therapeutic cells exceed the predetermined fluorescence threshold.

Also disclosed is a therapeutic cell product produced by the method including the steps of providing a quantity or population of cells, expanding the cells in tissue culture, and treating the quantity of therapeutic cells *in vitro* with an α1,3-fucosyltransferase and the fucose donor GDP-fucose, wherein the majority of the treated therapeutic cells have enhanced binding to P-selectin (or E-selectin) as described herein, and cryopreserving the cells. The quantity of cells may be derived from, for example but not by way of limitation, bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth, though they may be derived from cells grown in tissue culture or are cells derived from embryonic stem (ES) cells or induced pluripotent stem (iPS) cells. The therapeutic cells may also be any combination of the above.

In one embodiment the method comprises providing a quantity or population of therapeutic cells which lack or have reduced expression (less than the normal level of expression of CD38) of surface protein CD38, and treating the quantity or population of therapeutic cells *in vitro* with an α1,3-fucosyltransferase and the fucose donor GDP-fucose, wherein the therapeutic cells so treated have enhanced binding to P-selectin or E-selectin over the untreated therapeutic cells. Furthermore, the untreated therapeutic cells are typically characterized as predominantly comprising PSGL-1, CD44 and/or other selectin ligands that do not adequately bind to P-selectin or E-selectin or the therapeutic cells may lack expression of any selectin ligands. The PSGL-1 or other selectin ligands that occur on the therapeutic cells lack or have reduced numbers of fucosylated glycans, such as O-glycans, and may for example, have PSGL-1 which have core-2 O-glycans that comprise NeuAcα2,3Galβ1,4GlcNAc but that lack a fucose in α1,3 linkage to the GlcNAc. The therapeutic cells, in their untreated state prior to fucosylation, have reduced homing ability to bone marrow or to other desired sites that express selectins. In one particular, non-limiting embodiment, the therapeutic cells are derived from the list comprised of bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth, though they may be derived from cells grown derived from embryonic stem (ES) cells or induced pluripotent stem (iPS) cells, as long as they are characterized as needing, or benefiting from, further fucosylation to enhance their bone marrow homing ability. In the methods contemplated herein, the α1,3-fucosyltransferase may be for example α1,3-fucosyltransferase IV, α1,3-fucosyltransferase VI, or α1,3-fucosyltransferase VII. The fucose donor is GDP-fucose.

Also disclosed is a composition of treated therapeutic cells that comprise a cell population grown under cGMP-compliant conditions, wherein the treated cells comprise PSGL-1 or other selectin ligands that are properly fucosylated (e.g., comprises sialyl Lewis X) and that are able to bind to P-selectin (or E-selectin). The treated therapeutic cells may be disposed in a pharmaceutically acceptable carrier or vehicle for storage or administration to a patient. The treated therapeutic cells are cryopreserved for storage prior to administration to a patient.

In a particular, non-limiting embodiment, the therapeutic cells are selected from the list comprised of cord blood hematopoietic cells expanded under cGMP-compliant conditions, bone marrow-derived cells expanded under cGMP-compliant conditions, cord blood-derived cells expanded under cGMP-compliant conditions, mesenchymal stromal cells expanded under cGMP-compliant conditions, neural stem cells expanded under cGMP-compliant conditions, hepatocytes expanded under cGMP-compliant conditions, natural killer cells expanded under cGMP-compliant conditions and T cells expanded under cGMP-compliant conditions.

Disclosed are therapeutic cells that are expanded under cGMP-compliant conditions, cryopreserved under conditions that maintain optimal levels of fucosylation, and then thawed and fucosylated prior to delivery to a patient.

In one particular, non-limiting embodiment, the therapeutic cells are expanded under cGMP-compliant conditions, fucosylated and then cryopreserved under conditions that maintain optimal levels of fucosylation after the cells are thawed.

In a particular, non-limiting embodiment, the bone marrow-derived cells expanded under cGMP-compliant conditions are selected from the list comprised of AMR-001^{®} (Amorcyte, Inc., Allendale, NJ) ALD-301, ALD-201, ALD-401, bone marrow-derived cells expanded in the presence of the Notch ligand Delta1 and bone marrow-derived cells expanded in the presence of MSC.

In a particular, non-limiting embodiment, the cord blood-derived cells expanded under cGMP-compliant conditions are selected from the list comprised of NiCord^{®} (Gamida Cell Ltd., Jerusalem, Israel), Hemacord, ProHema, cord blood-derived cells expanded in the presence of the Notch ligand Delta1 and cord blood-derived cells expanded in the presence of MSC.

In a particular, non-limiting embodiment, the mesenchymal stromal cells expanded under cGMP-compliant conditions are selected from the list comprised of MULTISTEM^{®} (Athersys, Inc., Cleveland, OH), PROCHYMAL^{®} (Osiris Therapeutics, Inc., Columbia, MD), remestemcel-L, Mesenchymal Precursor Cells (MPCs), Dental Pulp Stem Cells (DPSCs), PLX cells, PLX-PAD, ALLOSTEM^{®} (Allosource, Centennial, CO), ASTROSTEM^{®} (Osiris Therapeutics, Inc., Columbia, MD), Ixmyelocel-T, MSC-NTF, NurOwn^{™} (Brainstorm Cell Therapeutics Inc., Hackensack, NJ), STEMEDYNE^{™}-MSC (Stemedica Cell Technologies Inc., San Diego, CA), STEMPEUCEL^{®} (Stempeudics Research, Bangalore, India), StempeuceICLI, StempeucelOA, HiQCell, Hearticellgram-AMI, REVASCOR^{®} (Mesoblast, Inc., Melbourne, Australia) CARDIOREL^{®} (Reliance Life Sciences, Navi Mumbai, India), CARTISTEM^{®} (Medipost, Rockville, MD), PNEUMOSTEM^{®} (Medipost, Rockville, MD), PROMOSTEM^{®} (Medipost, Rockville, MD), Homeo-GH, AC607, PDA001, SB623, CX601, AC607, Endometrial Regenerative Cells (ERC), and adipose-derived stem and regenerative cells (ADRCs) obtained with the CELUTION^{®} System (Cytori Therapeutics, Inc., San Diego, CA).

In a particular, non-limiting embodiment, the neural stem cells expanded under cGMP-compliant conditions are selected from the list comprised of NSI-566, HuCNS-SC^{®} (Stem Cells, Inc., Newark, CA), CTX0E03, ReN001, ReN009, STEMEDYNE^{™}-NSC (Stemedica Cell Technologies Inc., San Diego, CA), Q-CELLS^{®} (Q Therapeutics Inc., Salt Lake City, UT), TBX-01, TBX-02, RhinoCyte^{™} olfactory stem cells (RhinoCyte Inc., Louisville, KY), MOTORGRAFT^{®} (California Stem Cell, Inc., Irvine, CA), and CellBeads^{™} Neuro.

In a particular, non-limiting embodiment, the cardiac-derived cells expanded under cGMP-compliant conditions are cardiac-derived stem cells (CDCs).

In a particular non-limiting embodiment, the liver cells expanded under cGMP-compliant conditions are hpSC-derived hepatocytes, Heterologous Human Adult Liver Progenitor Cells (HHALPC), hLEC, and PROMETHERA^{®} HepaStem (Promethera Biosicences SA/NV, Belgium).

Also disclosed is a composition of treated therapeutic cells that comprises a population of human HSPC expanded under cGMP-compliant conditions having enhanced binding to P-selectin (or E-selectin). Enhanced binding to P-selectin (or E-selectin) is defined as at least 10% of the treated HSPC having fluorescence in a P-selectin binding assay (or E-selectin binding assay, respectively) which is greater than a predetermined fluorescence threshold. At least 25% of the treated HSPC exceed the predetermined fluorescence threshold. At least 50% of the treated HSPC exceed the predetermined fluorescence threshold. At least 75% of the treated HSPC exceed the predetermined fluorescence threshold. At least 90% of the treated HSPC exceed the predetermined fluorescence threshold. At least 95% of the treated HSPC exceed the predetermined fluorescence threshold. The composition of human HSPC may be disposed in a pharmaceutically-acceptable carrier or vehicle for storage or for administration to a subject.

Also disclosed is a composition of treated therapeutic cells that comprises a population of human MSC expanded under cGMP-compliant conditions having enhanced binding to P-selectin (or E-selectin). Enhanced binding to P-selectin (or E-selectin) is defined as at least 10% of the treated MSC having fluorescence in a P-selectin binding assay (or E-selectin binding assay, respectively) which is greater than a predetermined fluorescence threshold. At least 25% of the treated MSC exceed the predetermined fluorescence threshold. At least 50% of the treated MSC exceed the predetermined fluorescence threshold. At least 75% of the treated MSC exceed the predetermined fluorescence threshold. At least 90% of the treated MSC exceed the predetermined fluorescence threshold. At least 95% of the treated MSC exceed the predetermined fluorescence threshold. The composition of human MSC may be disposed in a pharmaceutically-acceptable carrier or vehicle for storage or for administration to a subject.

Also disclosed is a composition of treated therapeutic cells that comprises a population of human neural stem cells expanded under cGMP-compliant conditions having enhanced binding to P-selectin (or E-selectin). Enhanced binding to P-selectin (or E-selectin) is defined as at least 10% of the treated neural stem cells having fluorescence in a P-selectin binding assay (or E-selectin binding assay, respectively) which is greater than a predetermined fluorescence threshold. At least 25% of the treated neural stem cells exceed the predetermined fluorescence threshold. At least 50% of the treated neural stem cells exceed the predetermined fluorescence threshold. At least 75% of the treated neural stem cells exceed the predetermined fluorescence threshold. At least 90% of the treated neural stem cells exceed the predetermined fluorescence threshold. At least 95% of the treated neural stem cells exceed the predetermined fluorescence threshold. The composition of human neural stem cells may be disposed in a pharmaceutically-acceptable carrier or vehicle for storage or for administration to a subject.

Also disclosed is a composition of treated therapeutic cells that comprises a population of human hepatocytes expanded under cGMP-compliant conditions having enhanced binding to P-selectin (or E-selectin). Enhanced binding to P-selectin (or E-selectin) is defined as at least 10% of the treated hepatocytes having fluorescence in a P-selectin binding assay (or E-selectin binding assay, respectively) which is greater than a predetermined fluorescence threshold. At least 25% of the treated hepatocytes exceed the predetermined fluorescence threshold. At least 50% of the treated hepatocytes exceed the predetermined fluorescence threshold. At least 75% of the treated hepatocytes exceed the predetermined fluorescence threshold. At least 90% of the treated hepatocytes exceed the predetermined fluorescence threshold. At least 95% of the treated hepatocytes exceed the predetermined fluorescence threshold. The composition of human hepatocytes may be disposed in a pharmaceutically-acceptable carrier or vehicle for storage or for administration to a subject.

Also disclosed is a composition of treated therapeutic cells that comprises a population of human NK cells expanded under cGMP-compliant conditions having enhanced binding to P-selectin (or E-selectin). Enhanced binding to P-selectin (or E-selectin) is defined as at least 10% of the treated NK cells having fluorescence in a P-selectin binding assay (or E-selectin binding assay, respectively) which is greater than a predetermined fluorescence threshold. At least 25% of the treated NK cells exceed the predetermined fluorescence threshold. At least 50% of the treated NK cells exceed the predetermined fluorescence threshold. At least 75% of the treated NK cells exceed the predetermined fluorescence threshold. At least 90% of the treated NK cells exceed the predetermined fluorescence threshold. At least 95% of the treated NK cells exceed the predetermined fluorescence threshold. The composition of human NK cells may be disposed in a pharmaceutically-acceptable carrier or vehicle for storage or for administration to a subject.

Also disclosed is a composition of treated therapeutic cells that comprises a population of human T cells (such as, but not limited to, regulatory T cells and cytotoxic T cells (for example, but not by way of limitation, CD8+ cytotoxic T cells)) expanded under cGMP-compliant conditions having enhanced binding to P-selectin (or E-selectin). Enhanced binding to P-selectin (or E-selectin) is defined as at least 10% of the treated T cells having fluorescence in a P-selectin binding assay (or E-selectin binding assay, respectively) which is greater than a predetermined fluorescence threshold. At least 25% of the treated T cells exceed the predetermined fluorescence threshold. At least 50% of the treated T cells exceed the predetermined fluorescence threshold. At least 75% of the treated T cells exceed the predetermined fluorescence threshold. At least 90% of the treated T cells exceed the predetermined fluorescence threshold. At least 95% of the treated T cells exceed the predetermined fluorescence threshold. The composition of human T cells may be disposed in a pharmaceutically-acceptable carrier or vehicle for storage or for administration to a subject.

The predetermined fluorescence threshold is determined by first obtaining a sample of therapeutic cells. This control (baseline) sample of therapeutic cells is assayed using the P-selectin binding assay (or E-selectin binding assay) described elsewhere herein, or by any other P-selectin fluorescence binding assay (or E-selectin binding assay, respectively) known in the art or by staining with the HECA-452 antibody. P-selectin (or E-selectin or HECA-452) binding fluorescence levels are measured for the therapeutic cells in the control (baseline) sample. A fluorescence value can be selected that exceeds the P-selectin (or E-selectin or HECA-452) binding fluorescence levels of at least 95% of the therapeutic cells in the control sample. The selected fluorescence value is designated as the predetermined fluorescence threshold against which is compared the P-selectin (or E-selectin or HECA-452) binding fluorescence of the treated (i.e., fucosylated) therapeutic cells.

Also disclosed is a therapeutic cell product produced by the method of providing a quantity or population of therapeutic cells and treating the quantity of therapeutic cells *in vitro* with an α1,3-fucosyltransferase and the fucose donor GDP-fucose, wherein the majority of the treated therapeutic cells bind to P-selectin (or E-selectin or HECA-452). The quantity of therapeutic cells may be derived from bone marrow, but may be derived from cord blood, umbilical cord, peripheral blood, lymphoid tissue, adipose tissue, neural tissue, muscle, placenta, amniotic fluid, endometrium, liver or they may be derived from cells derived from embryonic stem (ES) cells or induced pluripotent stem (iPS) cells. The therapeutic cells may also be any combination of the above.

In general, a method of manufacture of therapeutic cells under cGMP conditions wherein non-functional or suboptimally functional PSGL-1 or other selectin ligands expressed on therapeutic cells are modified by *in vitro* α1,3-fucosylation technology to correct the homing defect, which improves their use in cell therapy, is disclosed.

As explained previously, therapeutic cells can express PSGL-1 or other selectin ligands, yet a significant amount do not bind to P-selectin (or E-selectin) or bind only low amounts of P-selectin (or E-selectin, respectively). PSGL-1 is a homodimeric mucin expressed on almost all leukocytes including CD34+ cells. To be functional, i.e., able to bind to P-selectin or E-selectin, PSGL-1 requires several post-translational modifications leading to formation of an sLex group thereon, including α1,3-fucosylation. Insufficient α1,3-fucosylation, for example, results in impaired ability of naive T cells to interact with vascular selecting. It has been discovered that the inability of therapeutic cells to bind to the P-selectin or E-selectin adhesion molecules can be corrected with *ex vivo* fucosylation after expansion under cGMP conditions, either before or after cryopreservation. This is a surprising finding given the number of adhesion molecules that are down-regulated with expansion (e.g., Kallinikou et al., Menard et al., Feneke et al.) and with cryopreservation (e.g., Faint et al., Koenigsmann et al., Hattori et al., Campbell et al., Aoyagi et al., Mareschi et al., and Neuhuber et al.).

The treatment of therapeutic cells *in vitro* with an α1,3-fucosyltransferase and the fucose donor GDP-fucose (e.g., FT-VI or FT-VII together with GDP-fucose), which also catalyzes the synthesis of the sLex structure, will increase fucosylation of PSGL-1 or other selectin ligands and thereby correct the homing defect of the therapeutic cells even after large-scale expansion in cGMP cultures. The fucosylated cells are cryopreserved and retain their fucosylation levels after thaw.

Fucosyltransferases that are able to transfer fucose in α1,3 linkage to GlcNAc are well known in the art. Several are available commercially, for example from R&D Systems (Minneapolis, MN). Further, at least eight different types of α1,3-fucosyltransferases (FTIII-VII) are encoded by the human genome. These include: the Lewis enzyme (FTIII), which can transfer fucose either α (1,3) or α (1,4) to Galβ4GlcNAc or Galβ3GlcNAc respectively (Kukowska-Latallo et al. (1990) Genes Dev., 4:1288); FTIV, which forms α (1,3) linkages, which does not prefer sialylated precursors (Goelz, et al. (1989) Cell, 63:1349; Lowe, et al. (1991) J. Biol. Chem., 266:17467); FTV (Weston, et al. (1992) J. Biol. Chem., 267:4152) and FTVI (Weston, et al. (1992) J. Biol. Chem., 267:24575) which form α(1,3) linkages, which can fucosylate either sialylated or nonsialylated precursors, and FTVII (Sasaki, et al. (1994) J. Biol. Chem., 269:14730; Natsuka, et al. (1994) J. Biol. Chem., 269:16789), which can fucosylate only sialylated precursors. FTIX preferentially transfers fucose to the GlcNAc residue at the nonreducing terminal end of the polylactosamine chain, resulting in the terminal Lex structure, whereas the other α1,3FUTs preferentially transfer a Fuc to the GlcNAc residue at the penultimate position, resulting in the internal Lex structure (Nishihara et al. (1999) FEBS Lett., 462:289-294). FTX and FTXI link alpha-I-fucose onto conalbumin glycopeptides and biantennary N-glycan acceptors but not onto short lactosaminyl acceptor substrates as do classical monoexonic alpha1,3-fucosyltransferases (Mollicone et al. (2009) J Biol Chem., 284:4723-38).

Sequence information for FTIII is disclosed by GC19M005843; FTIV by GC11P094277; FTV by GC19M005865; FTVI by GC19M005830; FTVII by GC09M139924, FTIX by GC06P096463, FTX by GC08M033286 and FTXI by GC10P075532 (GeneCards^{®} (Weizmann Institute of Science, Rehovot, Israel) is a searchable, integrated, database of human genes maintained by the Weizmann Institute that provides concise genomic related information on all known and predicted human genes as well as links to other databases). Other, non-human α1,3-fucosyltransferases available and known to those of ordinary skill in the art may be used, for example as shown in US Patent Nos. 6,399,337 and 6,461,835.

Human HSPC can be obtained for treatment with α1,3-fucosyltransferase, for example, by separation from the other cells in a source of umbilical cord blood, peripheral blood, or bone marrow. Various techniques well known in the art may be employed to obtain the HSPC including, but not limited to, density gradient separation, hypotonic lysis of red blood cells, centrifugal elutriation or separation with monoclonal antibodies using fluorescent-activated cell sorter (FACS) or magnetic bead isolation devices. Monoclonal antibodies are particularly useful for identifying markers (surface membrane proteins) associated with particular cell lineages and/or stages of differentiation. Antibodies such as anti-CD34 or anti-CD133 can be used to isolate HSPC under cGMP-compliant conditions, either by FACS or by magnetic bead using an instrument such as the CliniMACS^{®} System from Miltenyi Biotec Inc. (Bergish Gladbach, Germany). Alternatively, HSPC can be separated using a reagent such as ALDEFLUOR^{™} (STEMCELL Technologies, Inc., Vancouver, BC) that is oxidized in cells by aldehyde dehydrogenase (ALDH) into a charged fluorescent product that accumulates in cells and allows the separation of brightly fluorescent cells containing the HSPC by FACS. The separation techniques employed should maximize the retention of viability of the fraction to be collected. The particular technique employed will depend upon efficiency of separation, cytotoxicity of the methodology, ease and speed of performance, and necessity for sophisticated equipment and/or technical skill.

Once isolated, HSPC can be expanded using a variety of cGMP expansion protocols known in the art (see Tung et al. (2010) Best Pract Res Clin Haematol., 23:245-57 for review). Cells can be grown in tissue culture medium containing a cocktail of factors including, but not limited to, one or more from the list of factors comprised of erythropoietin, kit ligand, G-CSF, GM-CSF, IL-6, IL-11, thrombopoietin, flt ligand, FGF-1, angiopoietin-like 5, insulin-like growth factor binding protein 2 (IGFBP2), notch ligand delta 1, PIXY321, prostaglandin E2, aryl hydrocarbon nuclear receptor protein antagonists such as SR1, and tetraethylenepentamine (TEPA). Cells can also be expanded in co-cultures with MSC, which are thought to exert a favorable environment for the expansion of HSPC. Expansion under cGMP conditions can be conducted in tissue culture medium containing FBS, but it may be preferable to avoid xenogeneic serum and use serum-free media such as STEMLINE^{®} Medium (StemLine Therapeutics, Inc., New York, NY), CellGro^{®} Medium (MediaTech, Inc., Manassas, VA) QBSF-60, and the like. In certain embodiments, cells are expanded for 5 - 21 days prior to fucosylation and infusion into a patient. The fucosylation procedure used is described below.

Human MSC can be obtained for treatment with α1,3-fucosyltransferase, for example, by separation from the other cells in a source of bone marrow, umbilical cord blood, Wharton's jelly, adipose tissue, menstrual fluid, amniotic fluid or placenta. The source of cells can be autologous, allogeneic or xenogeneic. MSC can be obtained from cultures of embryonic stem cells or induced pluripotent stem cells. Various techniques known in the art may be employed to obtain the MSC depending on the source. For MSC derived from sources in which the MSC are trapped in a matrix, including but not limited to Wharton's jelly, adipose tissue and placenta, the MSC can be released by treatment with proteolytic enzymes including, but not limited to, collagenase, hyaluronidase, trypsin and dispase. Once MSC are isolated in a mixture of single cells they can be separated from the other cell types by methods known in the art including, but not limited to, adherence to plastic, density gradient separation, hypotonic lysis of red blood cells, centrifugal elutriation, binding to non-woven fibers as in the Bone Marrow MSC Separation Device from Kaneka, or separation with monoclonal antibodies using a fluorescent-activated cell sorter (FACS) or magnetic bead isolation devices such as the CliniMACS^{®} System from Miltenyi Biotec Inc. (Bergish Gladbach, Germany). Monoclonal antibodies useful for such separation include, but are not limited to, anti-NGF-R, anti-PDGF-R, anti-EGF-R, anti-IGF-R, anti-CD29, anti-CD49a, anti-CD56, anti-CD63, anti-CD73, anti-CD105, anti-CD106, anti-CD140b, anti-CD146, anti-CD271, anti-MSCA-1, anti-SSEA4, anti-STRO-1 and anti-STRO-3. The separation techniques employed should maximize the retention of viability of the fraction to be collected. The particular technique employed will depend upon efficiency of separation, cytotoxicity of the methodology, ease and speed of performance, and necessity for sophisticated equipment and/or technical skill.

Once isolated, MSC are grown in expansion cultures under cGMP conditions using methods known in the art. MSC can be grown in medium containing FBS but can also be grown in medium containing human platelet lysate instead of FBS or in serum-free media such as STEMPRO^{®} MSC SFM (Thermo Fisher Scientific Inc., Carlsbad, CA), STEMLINE^{®} Mesenchymal Stem Cell Expansion Medium (StemLine Therapeutics, Inc., New York, NY), CellGro^{®} MSC Medium (MediaTech, Inc., Manassas, VA), and the like. Cells are seeded at 5,000 - 5,000,000/cm², and non-adherent cells are removed by washing. Cells are typically passaged at 50 - 100% confluence after 7 - 28 days. After passage, cells may be expanded in tissue culture flasks, cell factories, roller bottles or bioreactors, including packed bed bioreactors that use beads, porous structures, fibers, non-woven fibers or hollow fibers as the substrate for cell growth. MSC can be safely passaged as many as 25 times but in certain embodiments are harvested after 3 - 8 passages, fucosylated, and cryopreserved. The methods for fucosylation and for cryopreservation are discussed below.

Human NSC can be obtained for treatment with α1,3-fucosyltransferase, for example, by separation from the other cells in cadaveric brain tissue. The source of cells can be autologous, allogeneic or xenogeneic. NSC can be obtained from cultures of embryonic stem cells or induced pluripotent stem cells. Various techniques known in the art may be employed to obtain the NSC. Mechanical disaggregation can be used and/or the cells can be released by treatment with proteolytic enzymes including, but not limited to, collagenase, hyaluronidase, trypsin and dispase. Once the NSC are isolated in a mixture of single cells they can be separated from the other cell types by methods known in the art including, but not limited to, adherence to plastic, density gradient separation, centrifugal elutriation, or separation with monoclonal antibodies using panning, a fluorescent-activated cell sorter (FACS) or magnetic bead isolation devices such as the CliniMACS^{®} System from Miltenyi Biotec Inc. (Bergish Gladbach, Germany). Monoclonal antibodies useful for such separation include, but are not limited to, anti-Integrin α1β5, anti-CD15, anti-CD24, anti-CD33, anti-CXCR4, anti-EGFR, anti-Notch1 and anti-PSA-NCAM. The separation techniques employed should maximize the retention of viability of the fraction to be collected. The particular technique employed will depend upon efficiency of separation, cytotoxicity of the methodology, ease and speed of performance, and necessity for sophisticated equipment and/or technical skill.

Once isolated, NSCs are grown in expansion cultures under cGMP conditions using methods known in the art. NSC can be grown in medium containing FBS but can also be grown in medium containing human platelet lysate instead or in serum-free media such as STEMPRO^{®} MSC SFM (Thermo Fisher Scientific Inc., Carlsbad, CA), STEMLINE^{®} Mesenchymal Stem Cell Expansion Medium (StemLine Therapeutics, Inc., New York, NY), CellGro^{®} MSC Medium (MediaTech, Inc., Manassas, VA), and the like. Cells are seeded at 5,000 - 5,000,000/cm², and non-adherent cells are removed by washing. Cells are typically passaged at 50 - 100% confluence after 7 - 28 days. After passage, cells may be expanded in tissue culture flasks, cell factories, roller bottles or bioreactors, including packed bed bioreactors that use beads, porous structures, fibers, non-woven fibers or hollow fibers as the substrate for cell growth. MSC can be safely passaged as many as 25 times but in certain embodiments are harvested after 6 - 8 passages, fucosylated and either delivered to the patient or cryopreserved. Alternatively, NSC can be conditionally immortalized as, for example, with the c-mycER^{TAM} transgene in CTX0E03 cells. The methods for fucosylation and for cryopreservation are discussed below.

The fucosylation process is conducted under cGMP conditions. This requires that all reagents used be produced under cGMP conditions. For example, cDNA for FTVI can be obtained by methods known in the art, such as using PCR using to amplify the gene from a cDNA libray such as the Clontech Quick-Clone II human Lung cDNA library. Once obtained, the cDNA can be cloned into a cloning vector such as the Invitrogen PCR-Blunt Topo PCR cloning vector. DNA sequencing can be used to verify that the correct sequence was cloned by comparing the obtained sequence with DNA databases. The cDNA can then be cloned into a vector containing an affinity tag such as the pCDNA 3.1 (+) from Invitrogen containing the HPC4 epitope and then subcloned into an expression vector such as the Lonza pEE14.1 expression vector (Lonza Walkersville, Inc., Walkersville, MD). The Lonza pEE14.1 uses glutamine synthetase (GS) for high-level gene amplification, which typically requires only a single round of selection for amplification to achieve maximal expression levels. Cells such as CHO-K1 cells (ATCC: CCL-61) can be transfected with the construct containing FTVI cDNA and a HPC4 tag at its N-terminus. After amplification, clones expressing FT-VI/HPC4 at high levels can be selected.

If CHO cells are chosen for protein production, then methods known in the art can be utilized to make master and working cell banks for cGMP production of protein.

Alternative methods of protein production known in the art can be used as well including, but not limited to, expression in prokaryotes such as bacteria like *E. coli,* yeast like *Pichia pastoris,* insect cells such as insect cells via baculovirus and other mammalian cell lines such as NSO, HEK, and the like. Other affinity tags known in the art can be used including, but not limited to, FLAG-tag, V5-tag, c-myc-tag, His-tag, HA-tag and the like. Alternatively, proteins can be expressed in the absence of a tag and purified using various chromatography techniques known in the art including, but not limited to, ion exchange, gel filtration, reverse-phase HPLC and the like. Combinations of affinity purification and chromatography can also be used. Similar techniques can be used for cGMP production of any α1,3-fucosyltransferase. It is not necessary to express the full-length α1,3-fucosyltransferase protein; truncated proteins as well as proteins engineered by methods known in the art to improve stability, specificity or activity can also be used for *ex vivo* fucosylation of therapeutic cells as long as they retain enzymatic activity. The α1,3-fucosyltransferase protein can be used as a free enzyme in solution or can be immobilized to a substrate such as a bead or column in order to facilitate removal of enzyme from the therapeutic cells.

### EXAMPLES

Examples are provided hereinbelow. However, the invention is to be understood to not be limited in its application to the specific experimentation, results and laboratory procedures. Rather, the Examples are simply provided as one of various embodiments and are meant to be exemplary, not exhaustive.

### Example 1

Different cells types require different fucosylation conditions. As shown below, neural stem cells could not be fucosylated with FTVI but were fully fucosylated with FTVII (Experiment D); similarly, B (CD19+), T (CD3+ or CD4+), and CD38+ cells were fucosylated with FTVII but not FTVI (Example 5, described herein after). Other cell types were fucosylated equally with either enzyme. It is not possible to determine *a priori* what enzymes will fucosylate which cell type.

In order to compare the effects of *ex vivo* fucosylation on different cell types, recombinant FTVI produced in CHO cells was manufactured at Aragen Bioscience (Morgan Hill, CA; final concentration 1100 µg/mL) and FTVII produced in a mouse lymphocyte line was obtained from Kyowa Hakko Kirin (Japan, final concentration 150 µg/mL). Frozen human umbilical cord bloods were purchased from the San Diego Blood Bank. Human mesenchymal stem cells and human CD34+ cord blood cells were purchased from Lonza (Lonza Walkersville, Inc., Walkersville, MD). Fresh human neural stem cells were obtained from the laboratory of Evan Snyder at Sanford/Burnham. Endothelial progenitor cells (EPCs) were a gift from Dr. Joyce Bischoff (Vascular Biology Program and Department of Surgery, Children's Hospital, Harvard Medical School, Boston, MA). Human amniotic stem cell lines were from the laboratory of Shay Soker at Wake Forest University. Human adipose-derived stem cells were from the laboratory of Brian Johnstone, Indiana University. The cells were grown in EGM-2, 20% heat-inactivated fetal bovine serum, 1% GPS , and all growth factors in EGM-2 bullet kit from Lonza (#CC-3162; Lonza Walkersville, Inc., Walkersville, MD), excluding hydrocortisone, in a 5% CO₂, 37°C incubator. Cells were treated at 10⁶ cells/mlLfor 30 minutes at room temperature with 1 mM GDP β-fucose (EMD Biosciences, San Diego, CA.) in Phosphate Buffered Saline (PBS) containing 1% human serum albumin (HSA, Baxter Healthcare Corp., Westlake Village, CA.) and in the previously optimized concentrations of 100 mU/mL FT-VI, or 75 µg/mL FT-VII. Untreated cells were incubated as above except no enzyme was added. Fucosylation levels were determine by flow cytometry using HECA-452 antibody (BD Biosciences), a directly conjugated (FITC), rat IgM antibody that reacts against a fucosylated (sialyl Lewis X (sLeX)-modified) form of P-selectin glycoprotein ligand (PSGL)-1 (CD162), also known as cutaneous lymphocyte antigen (CLA). Other antibodies to CD antigens were also obtained from BD Biosciences.

All of the following experiments have been replicated with similar if not nearly identical results in the replicate.

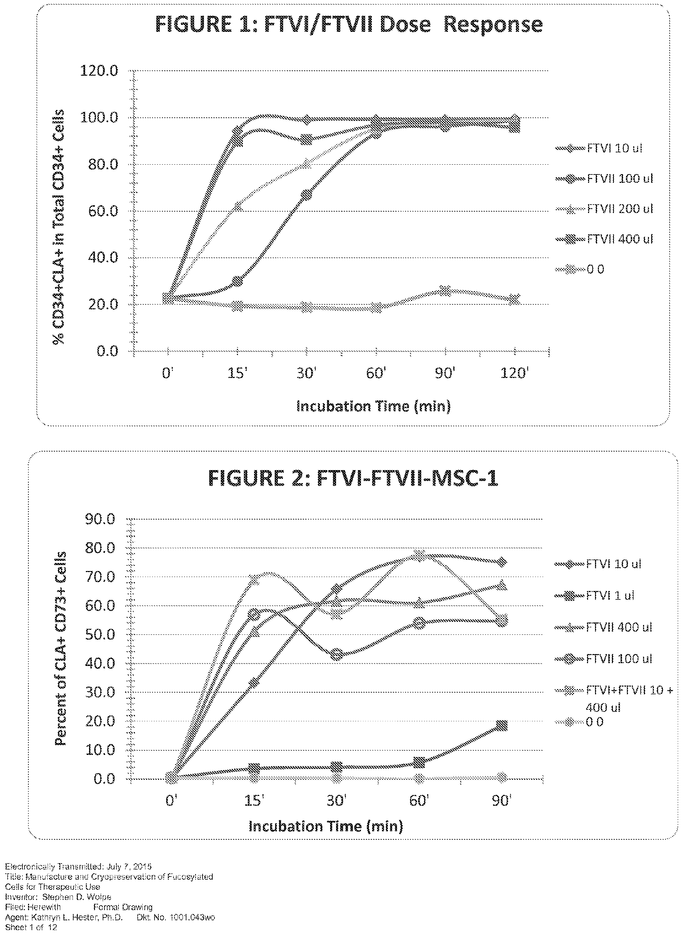

Figure 1 illustrates a comparative analysis of FTVI (10 µl = 11 µg/mL) versus FTVII (100 µl=15 µg/mL, 200 µl = 30 µg/mL and 400 µl = 60 µg/mL) on the kinetics of cell surface fucosylation (%CLA-FITC) using mononuclear cells from thawed human cord blood incubated for the indicated time points.

### Example 2

Figure 2 illustrates a comparative analysis of FTVI (11 and 1.1 µg) versus FTVII (15 and 60 µg) on the kinetics of fucosylation (%CLA-FITC) using human mesenchymal stem cells (MSCs). The same conditions as described for Example 1 were used.

Figure 2 illustrates that FTVII at both 100 µl (15µg) and 400 µl (60 µg) was able to achieve significant fucosylation of mesenchymal stem cells (MSCs) at the early time point (15 min), demonstrating that FTVII is more active at fucosylating and generating CLA sites than FTVI at 10 µl (11 µg). By 30 minutes, the differential effect of FTVII versus FTVI was no longer observed. Replicate results demonstrate that the maximal effect of fucosylation on MSCs was not significantly different between the two isoforms of FT and that the maximally achieved percent CLA expression was around 70% - 80%.

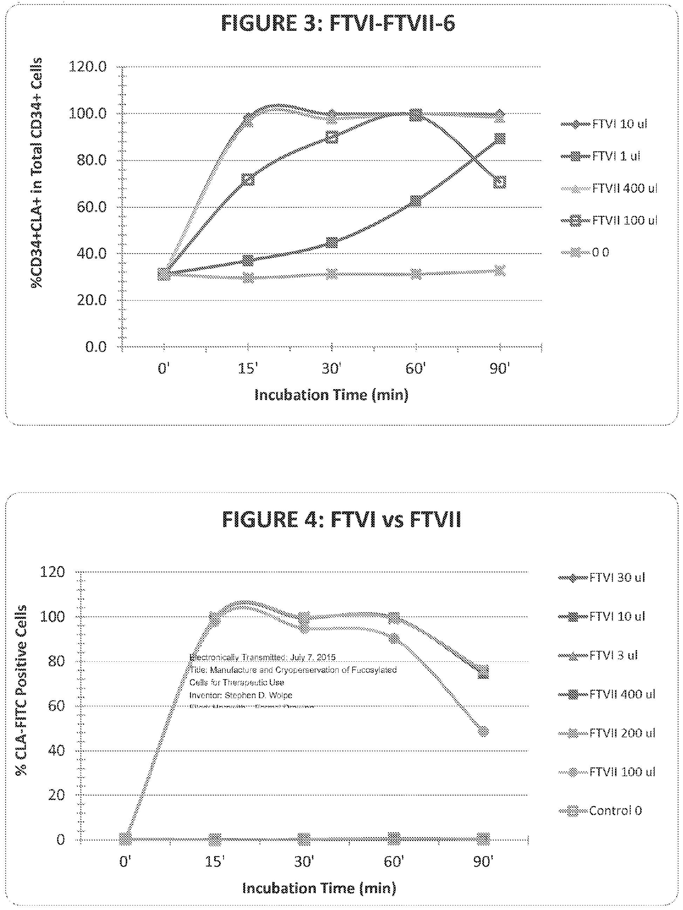

Figure 3 illustrates a comparative analysis of FTVI (11 and 1.1 µg) versus FTVII (15 and 60 µg) on the kinetics of fucosylation (%CLA-FITC) using purified cord blood-derived CD34+ cells. The same conditions as described in Example 1 were used.

Using purified CD34+ cells derived from cord blood, the results in Figure 3 parallel the results observed with a CB MNC preparation in Figure 1; that is, maximal % fucosylation was observed with FTVI at 10 µl (11 µg) and FTVII at 400 µl (60 µg) with a time dependent achieval of maximal effect at lower concentrations of each FT. The lower dose of FTVII (100 µl, 15 µg) achieved a greater level of fucosylation at the early time point of 15 minutes (70%) than was observed at the same time point in the MNC preparation in Figure 1 (30%).

Figure 4 illustrates a comparative analysis of FTVI (33, 11 and 3.3 µg) versus FTVII (15, 30 and 60 µg) on the kinetics of fucosylation (%CLA-FITC) using fresh cultured neural stem cells (NSCs). The same conditions as described in Example 1 were used.

The results in Figure 4 show no baseline level of fucosylation of a purified population of neural stem cells (NSCs). The Figure also shows that FTVI at a concentration of 10 µl (11 µg) and above (30 µl, 33 µg), which fully fucosylates CD34+ cells, was unable to change the baseline level of fucosylation. Only FTVII at both concentrations (100 µl and 400 µl) was able to fucosylate these cells, achieving maximal fucosylation at the earliest time point of 15 minutes.

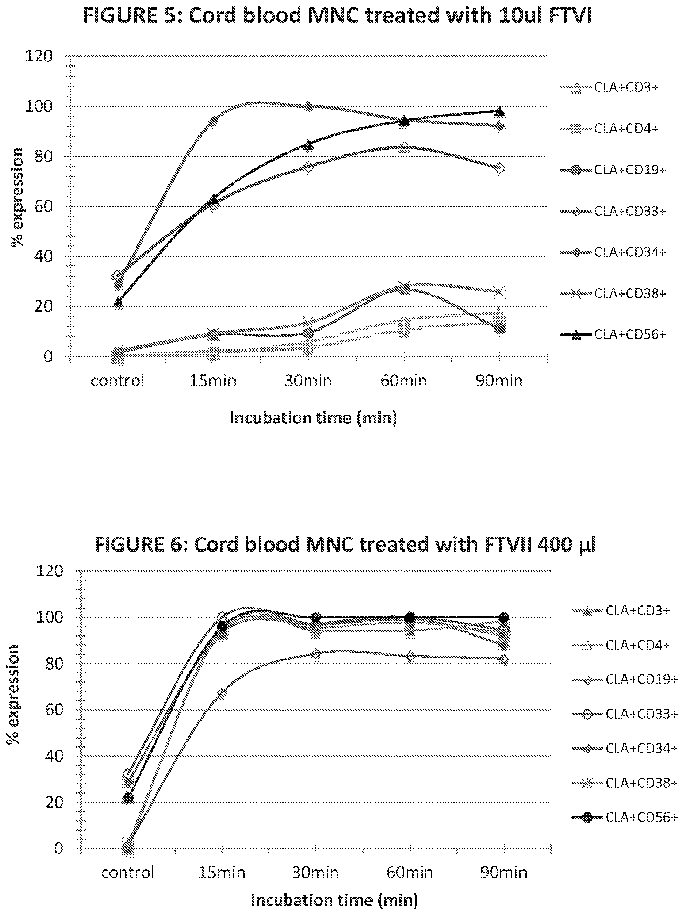

Figures 5 and 6 illustrate a comparative analysis of FTVI (11 µg; Figure 5) versus FTVII (60 µg; Figure 6) on the kinetics of fucosylation using human thawed cord blood-derived mononuclear cells. The same conditions as described in Example 1 were used.

The results above illustrate that FTVI (Figure 5) was able to fucosylate only select cells in the mixed population of cells from cord blood. Both B and T lymphocytes (CD3, CD4, CD19) were only modestly affected following incubation with FTVI at a dose (10 µl, 11 µg) that fully fucosylates CD34+, CD33+, and CD56 cells; similarly, CD38+ cells were only minimally fucosyated by FTVI. By contrast FTVII at 400 µl (60 µg; Figure 6) was able to achieve nearly 100% fucosylation of all cell types examined, including the various lymphocyte subpopulations in a cord blood mononuclear cell (MNC) preparation.

### Example 3

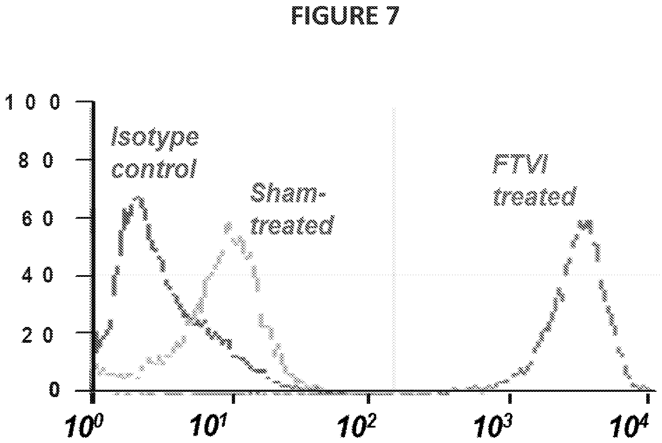

Figure 7 illustrates an analysis of the effects of FTVI treatment versus sham treatment on fucosylation of human endothelial progenitor cells (EPCs). The same conditions as described in Example 1 were used. All cells were fucosylated by *ex vivo* treatment with FTVI.

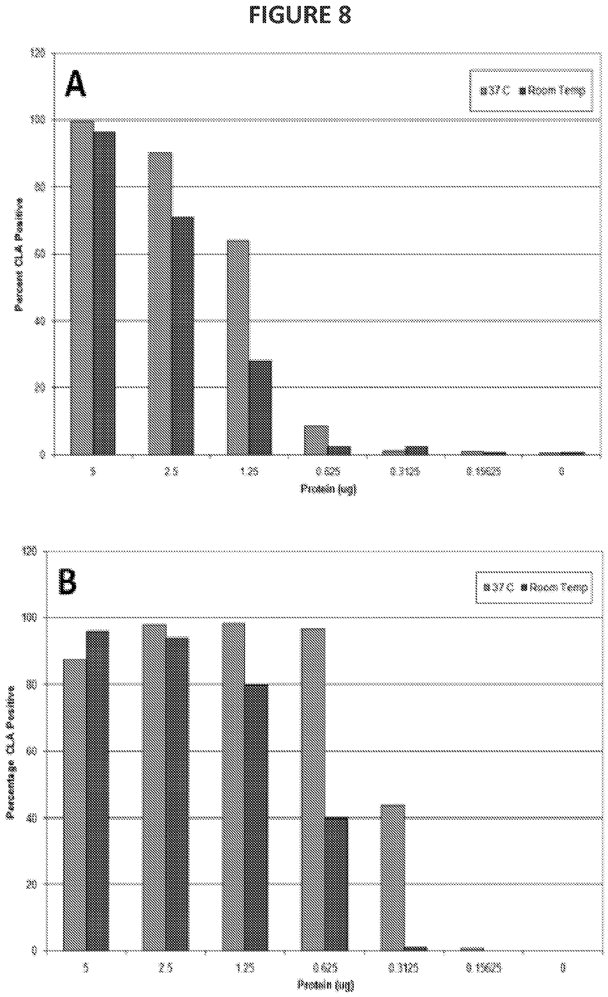

Figure 8 illustrates an analysis of the effects of FTVI treatment on fucosylation of human amniotic stem cells. The same conditions as described in Example 1 were used except that incubation at 37°C (blue lines) was also tested.

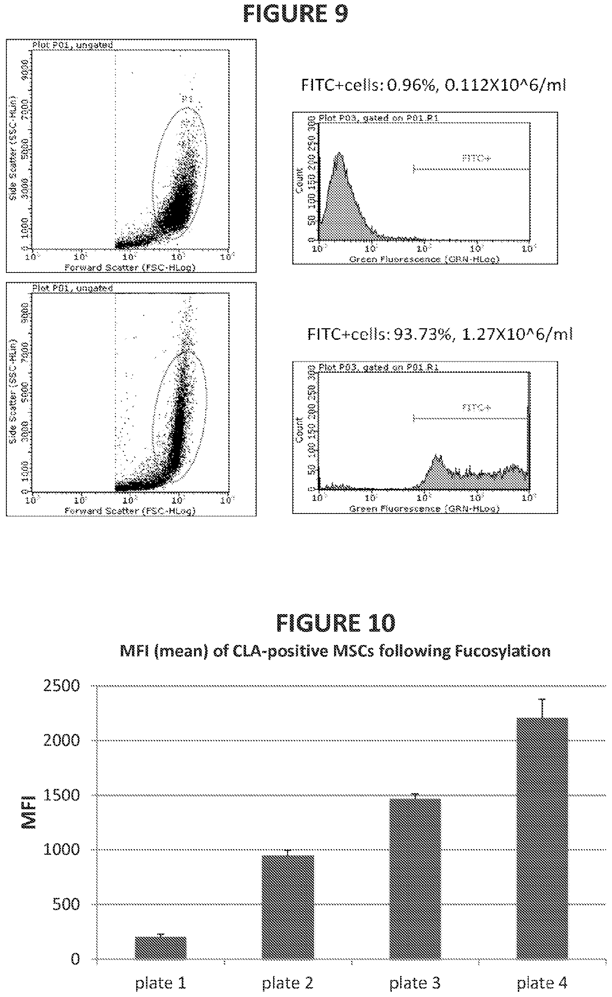

Figure 9 illustrates an analysis of the effects of FTVI treatment on fucosylation of human adipose-derived stem cells. The same conditions as described in Example 1 were used. As shown in this Figure, greater than 90% of adipose-derived stem cells were fucosylated by FTVI.

### Example 4

The experiments in this Example were conducted to test the stability of fucosylation of mesenchymal stem cells (MSC) following cryopreservation. Frozen aliquots of MSC were obtained from Lonza (Lonza Walkersville, Inc., Walkersville, MD) and defrosted. The cells were washed to remove cryoprotectant and then resuspended in Hank's basic salt solution (HBSS) plus 1% human serum albumin (HSA). One aliquot was used as control and the other fucosylated according to the following procedure: to MSCs in 800 µl of HBSS + 1% HSA containing MSCs, were added 100 µl HBSS + 1% HAS, 100 µl GDP-fucose (10 mM stock), and 10 µl of FTVI to start reaction, which was terminated by washing after 30 minutes. Control cells followed the same protocol but without added enzyme.

The cells were incubated at room temperature with gentle mixing for 45 minutes and then washed by centrifugation. The resulting pellet was resuspended in HBSS + 1% HAS. An aliquot of cells was removed for analysis by FACS using CLA-FITC as above and anti-CD73 as a specific MSC cell surface marker. Propidium iodide was used to measure viability.

The remaining cells were washed by adding 2 mL HBSS + 1% HSA and cryopreserved according to the following protocol: (1) the pelleted cells were in equal volumes (0.5 mL) of 100% FBS and 20% DMSO; and (2) cells were placed in a -70°C freezer for two hours, then transferred to liquid nitrogen.

The next day, the cells were thawed, washed by centrifugation and resuspended in 1.0 mL HBSS + 1%HSA and assayed by flow cytometry as above. Results of the assays are shown in Table 1.

As can be seen, fucosylation levels were maintained after cryopreservation, both in terms of percent of cells fucosylated and MFI, though there was a loss of cell viability.

| **TABLE 1** | | | | | |
|---|---|---|---|---|---|
| | **Before Freezing** | | **After freezing** | | |
| | **% CLA Positive** | **CLA-FITC MFI** | **% CLA Positive** | | **CLA-FITC MFI** |
| **Isotype Control** | 0.4 | 29.4 | 2.7 | | 33.8 |
| **Control Cells** | 0.5 | 38.8 | 0.6 | | 38.6 |
| **Fucosylated Cells** | 98.1 | 1054.6 | 96.8 | | 1018.3 |
| | | | | | |

| | **Before Freezing** | **After Fucosylation** | | **After freezing** | |
|---|---|---|---|---|---|
| **Cell count** | 1.04 x 10⁶/mL | 0.95 x 10⁶/mL | | 0.93 x 10⁶/mL | |
| **Viability** | 96.60% | 95.30% | | 87.50% | |

### Example 5

In order to compare the effects of fucosylation of human MSC either before or after trypsinization, the following experiment was conducted. Human MSC were expanded in serum-free media for 11 days, plated in 6-well plates, and allowed to adhere over several days. Serum-free media in Plates 1 - 3 was exchanged with the indicated media (Plate 1: Media + 10% FBS, Plate 2: serum-free media, Plate 3: HBSS), after which cells were exposed to TZ101 under conditions know to achieve maximal fucosylation and MFI. Cells from Plate 4 were suspended in HBSS before exposure to TZ101 to obtain the maximal level of expression of % CLA-FITC and MFI. Results are shown in Table 2 and are the average of values from two separate wells.

As shown in Figure 10, trypsinization prior to fucosylation resulted in higher MFI values than fucosylation of adherent cells under any conditions. Fucosylation of cells in HBSS resulted in higher MFI than fucosylation of cells in serum-free medium.

| **TABLE 2** | | | | |
|---|---|---|---|---|
| | **Fucosylation Condition** | **MSC** | **% CLA-FITC** | **MFI (mean)** |
| Plate 1 | Adherent - Media + 10%FBS | Control | 9 | 98 |
| | | Treated | 31 | 205 |
| Plate 2 | Adherent - Serum-free Media | Control | 1 | 333 |
| | | Treated | 91 | 944 |
| Plate 3 | Adherent - HBSS | Control | 1 | 359 |
| | | Treated | 96 | 1469 |
| Plate 4 | Suspension - HBSS | Control | 1 | 350 |
| | | Treated | 100 | 2208 |

### Example 6

MSC are grown and fucosylated under cGMP conditions. After written informed consent of the donor, 15 mL of bone marrow are harvested from the iliac crest. The bone marrow is seeded at 10⁵ nucleated cells/cm² onto a two level CellSTACK^{®} culture chamber (1272 cm², Corning, Acton, MA) in 300 mL of culture medium (αMEM (Life Technologies, Grand Island, NY) supplemented with 8% human platelet lysate (Mill Creek Life Sciences, Rochester, Minnesota). The entire medium is renewed twice weekly until cells reached confluence (end of P0). Then, cells are detached using trypsin (Hyclone), viable cells are counted, and cells reseeded at 10³ cells/cm² onto five two level CellSTACK^{®} culture chambers. After two weeks (end of P1), cells are detached by trypsinization, viable cells counted, and the process repeated twice (P2 and P3).

At the end of P3, cells are detached by trypsinization, washed in Hank's basic salt solution (HBSS) plus 1% recombinant human serum albumin (HSA) (InVitria, Ft. Collins, CO), and resuspended at a concentration of 10⁷/mL in HBSS plus 1% HSA. The cells are fucosylated by incubating with recombinant FTVI (0.01 mg/mL) plus 1 mM GDP-fucose (both sourced from America Stem Cell, San Diego, CA) for 30 minutes at room temperature, washed, and cryopreserved.

For cryopreservation, a total of 10 mL of MSCs at 10⁷ cells per mL are mixed with 10 mL of freeze mix consisting of 10% DMSO, 12% Pentastarch, and 8% Human Serum Albumin (HSA) in plasmalyte A and transferred into customized 20 mL FEP cryobags (AFC Kryosure VP-20f, Gaithersburg, MD). The cells are cryopreserved using a controlled rate freezer (Kryosave, Cryo Associates, Gaithersburg, MD) and stored in the vapor phase of a liquid nitrogen tank.

### Example 7

Twenty million 100 Gy-irradiated and washed Epstein-Barr virus-transformed lymphoblastoid (EBV-LCL) cells were co-cultured with 10⁶ magnetic bead-purified human natural killer (hNK) cells in upright 75 cm² tissue culture flasks in 15 mL of X-VIVO 20 (Lonza, Walkersville, MD), supplemented with 10% heat inactivated human AB serum (Gemini Bio-Products, West Sacramento, CA), 500 IU/mL rhIL-2 (50 ng/mL, Tecin^{™}, Hoffmann-La Roche Inc., Nutley, NJ), and 2 mM GlutaMAX-1 (Invitrogen, Carlsbad, CA) at 37°C and 6.5% CO₂. After five days of culture, half of the culture medium was replaced. Starting on day 7, NK cells were diluted to 0.6 × 10⁶ cells/mL with growth medium containing IL-2 every 24 - 72 hours for 14 days.

The phenotype of the NK cells was assessed by flow cytometry on a FACSCalibur^{™} flow cytometer (BD Biosciences, San Jose, CA) with the following anti-human monoclonal antibodies: anti-CD56-APC (clone B159), anti-CD16-FITC (clone 3G8), anti-CD3-PE (clone UCHT1), anti-CD25-PE (clone M-A251), anti-NKG2D-APC (clone 1D11), anti-CD244-PE (2B4, clone 2 69), anti-CD48-FITC (clone TÜ145), anti-CD11a/LFA-1-PE (clone G43-25B), anti-FasL-biotin (clone NOK-1), anti-perforin-FITC (clone δG9), CD158b-PE (KIR2DL2/3, clone CH-L) and anti-CLA (HECA)-FITC antibody; cell viability was determined by staining with Via-Probe^{™} (BD Bisociences, San Jose, CA)(7AAD). Intracellular staining was performed on cells that were permeabilized and fixed using BD Biosciences' Cytofix/Cytoperm^{™}. Above antibodies and reagents were purchased from BD Biosciences (San Diego, CA) and were used according to manufacturer's specifications. Anti-granzyme A-FITC (clone CB9), anti-granzyme B-PE (clone GB11), and anti-TRAIL-PE (clone RIK-2) were purchased from Abcam Inc. (Cambridge, MA). Anti-NKG2A-APC (CD94/CD159a, clone 131411) and anti-NKG2C-PE (CD94/CD159c, clone 134591) were purchased from R&D Systems (Minneapolis, MN). Anti-KIR3DL1-PE (clone DX9) was obtained from BioLegend Inc. (San Diego, CA). Cells were also stained with their corresponding isotype-matched control monoclonal antibodies.

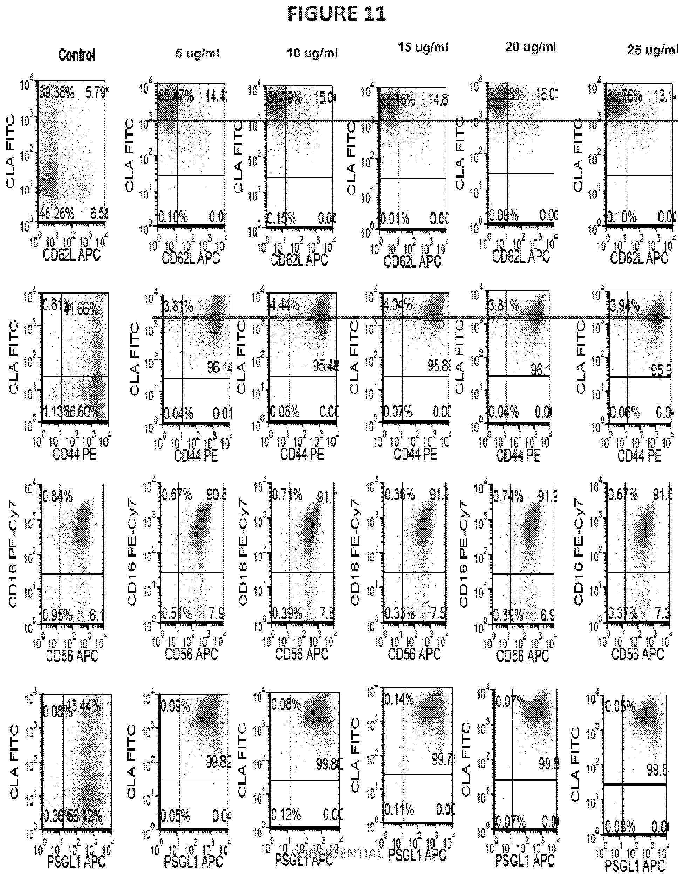

The results in Figure 11 show the fucosylation levels of human expanded hNK cells after incubation with TZ101 with varying concentrations of FTVI. While the maximal % of cells that achieved fucosylation (as reflected by reactivity with CLA-FITC) was observed at the lowest dose of FTVI (5 µg/mL) examined, maximal MFI was achieved at the higher FTVI doses of 20 -25 µg/mL (MFI for control = 36, at 5 µg/mL FTVI = 2033, at 10 µg/mL FTVI = 2097, at 15 µg/mL FTVI = 1943, at 20 µg/mL FTVI = 2205, and at 25 µg/mL FTVI = 2116). Other observations from the present Example include that there was no change in the phenotype, as reflected by stable levels of CD16 and CD56 staining, barely detectable levels of L-selectin, and high baseline levels of CD44 and PSGL on NK cells.

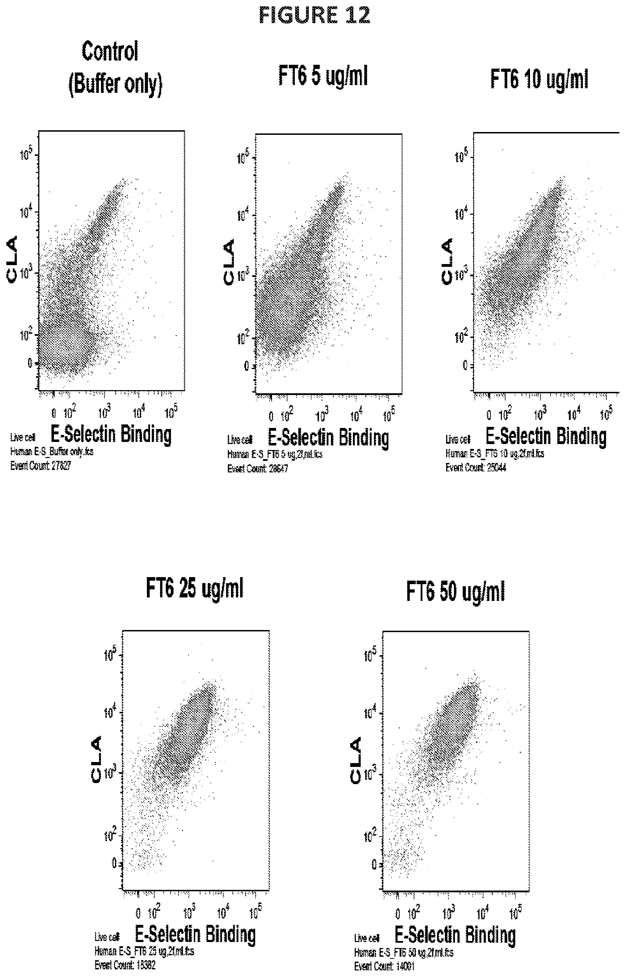

Fluid Phase Binding of NK Cells to E-selectin is Enhanced by Preincubation of NK Cells with TZ101. The effect of pretreatment of expanded hNK cells with TZ101 on the fluid-phase binding to E-selectin chimera was examined. Figure 12 illustrates a dose-response effect of FTVI on the binding of E-selectin to NK cells, with maximal binding achieved following incubation at a FTVI dose of 25 µg/mL, which correlates with the MFI results. Thus, all further studies were conducted using FTVI at 25 µg/mL.

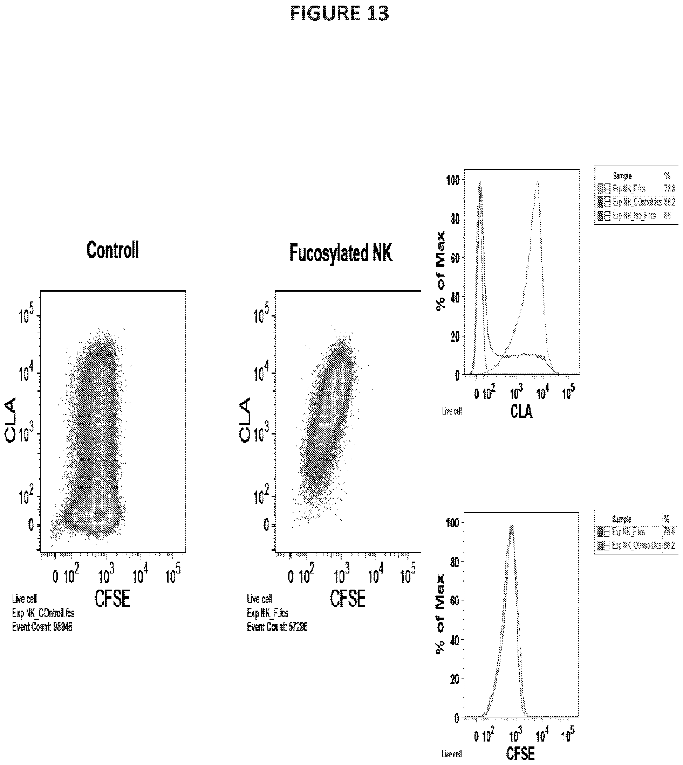

Stability of Fucosylation Achieved Following Incubation at Room Temperature. The results in Figure 13 show that hNK cells retain their fucosylation levels following incubation for 48 hours in tissue culture (FTVI at 25 µg/mL and GDP-fucose at 1mM). Furthermore, the data demonstrate that CD44 on NK cells may be the predominate site of action of FTVI in the enzymatically-mediated transfer of fucose to the tetrasaccharide, siLeX moiety decorating this cell surface glycoprotein.

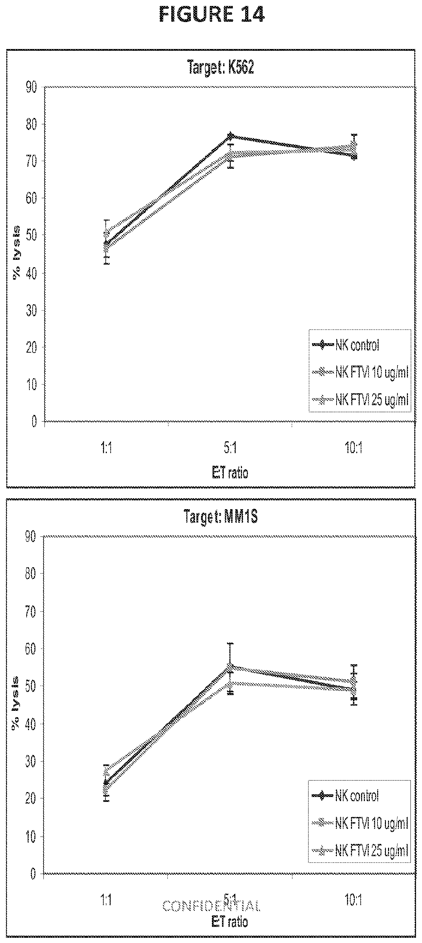

Cytotoxic Potency of NK Cells Maintained following Fucosylation. The results in Figure 14 demonstrate that fucosylated hNK Cells exhibit a cytotoxic profile *in vitro* that is similar to what is observed with control cells. In particular, incubation of either control or TZ101-treated (10 µg/mL or 25 µg/mL FTVI) NK cells, pre-activated by exposure to IL-2, exhibited potent cytotoxic effects against both K562 and MM1S (multiple myeloma) cells.

### Example 8

NK cells are manufactured and fucosylated under current good manufacturing practice (cGMP) conditions. All reagents used, including FTVI, are cGMP grade. 12 - 24 × 10⁶ magnetic bead-purified NK cells are combined with 120 - 240 × 10⁶ irradiated EBV-TM-LCL cells in 100 - 140 mL of medium containing rhIL-2 obtained from CellGenix Inc. (Portsmouth, New Hampshire) in Baxter 180 cm² 300 mL bags (Fenwal Lifecell, Baxter Healthcare Corporation, Deerfield, IL). Four to five days after the initiation of the culture, half of the medium is replaced. Two days later, the concentration of NK cells is adjusted to 10⁶ cells/mL using growth medium containing IL-2. Expanding cells are counted and diluted every 24 - 72 hours until day 28. A portion of the cells is cryopreserved in PlasmaLyte A medium (Baxter) supplemented with 4% human serum albumin (HSA, Talecris Biotherapeutics, Inc., Research Triangle Park, NC), 6% pentastarch (Hypoxyethylstarch, NIH PDS), 10 µg/mL DNase I (Pulmozyme, Genentech, Inc., South San Francisco, CA), 15 U/mL heparin (Abraxis Pharmaceutical Products, IL), and 5% DMSO at 20 - 50 × 10⁶ cells/mL per vial using a controlled-rate freezer followed by transfer to the vapor phase of a liquid nitrogen tank. After two weeks, the cells are thawed using thawing medium containing X-VIVO 20, 10% human AB serum, 4% HSA, and 10 U/mL heparin. Cells are thawed at 37°C, slowly diluted with 10 mL of thawing medium, and left at room temperature for 1 - 2 hours before being centrifuged to avoid cell breakage. Thawed cells are tested for fucosylation levels two hours following thawing, gating on viable cells using 7AAD staining. Fucosylation levels (as measured by MFI) should be observed to be ±10% of levels observed prior to cryopreservation.

### Example 9

Regulatory T cells (T_{regs}) were expanded from cord blood (CB). Cryopreserved CB units were thawed and washed in CliniMACS buffer (Miltenyi Biotec, Bergish Gladbach, Germany) containing 0.5% HSA (Baxter Healthcare, Westlake Village, CA) to yield CB mononuclear cells (MNC). CB MNC were then subjected to CD25+ cell enrichment using magnetic activated cell sorting (MACS) according to manufacturer's instructions (Miltenyi Biotec, Bergish Gladbach, Germany). Positively selected cells were co-cultured with CD3/28 co-expressing Dynabeads^{®} (ClinExVivo^{™} CD3/CD28, Invitrogen Dynal AS, Oslo, Norway) in a 1 cell: 3 bead ratio and resuspended at 1 x 10⁶ cells/mL in X-VIVO 15 medium (Cambrex BioScience, Walkersville, MD) supplemented with 10% human AB serum (Gemini Bio-Products, Sacramento, CA), 2 mM L-glutamine (Sigma, St. Louis, MO), 1% Penicillin-Streptomycin (Gibco/Invitrogen, Grand Island, NY)] and 200 IU/mL interleukin (IL)-2 (CHIRON Corporation, Emeryville, CA), in tissue culture flasks at 37°C in a 5% CO₂-in-air atmosphere.

The CB-derived CD25+ enriched T-cells were maintained at 1 × 10⁶ cells/mL by the addition of fresh medium and IL-2 (maintaining 200 IU/mL) every 48 - 72 hours. The average number of CD25+ cells isolated from one CB was 0.78 x 10⁶; after two weeks expansion, up to 400 x 10⁶ T_{reg} cells could be obtained.

Fucosylation was characterized by the presence of sLeX residues, as assessed by flow cytometry with antibody HECA-452 (BD Biosciences, San Jose, CA). A portion of the cells were removed pre- and post-fucosylation for flow staining with CLA, CD4, CD127, and CD25 antibodies.

*Ex vivo* fucosylation of expanded T_{reg} cells was performed on day 11 when the cultured cells were harvested and washed in PBS 1%HSA. The cells were then incubated with TZ101 (10 µg/mL of FTVI + 1 mM GDP-fucose) for 30 minutes at room temperature with occasional mixing, then washed and resuspended in PBS. A portion of cells was removed pre- and post-fucosylation for flow staining with CLA, CD4, CD127, and CD25 antibodies. The results are shown in Figure 15A and demonstrate that FTVI increased fucosylation levels on T_{reg} cells from 8.8% to 62%. In addition, fucosylated T_{regs} were able to suppress *in vitro* allo-mixed lymphocyte reaction (MLR) (Figure 15B).

### Example 10

Regulatory T cells (T_{regs}) are expanded from cord blood (CB) and then fucosylated, all under cGMP conditions. Cryopreserved CB units are thawed in a 37°C sterile saline bath using 10% dextran 40/5% human serum albumin as a wash solution. A MgCl₂/rHuDNAse/sodium citrate cocktail is used to prevent clumping prior to the immunomagnetic selection. Enrichment of CD25+ T_{reg} cells is accomplished by positive selection with directly conjugated anti-CD25 magnetic microbeads (Miltenyi Biotec, Bergish Gladbach, Germany) and a CliniMACS device (Miltenyi). After the column selection, CD25+ cells are suspended at a concentration of approximately 1 x 10⁶ cells/mL in X-VIVO 15 (Cambrex BioScience, Walkersville, Maryland, USA) supplemented with 10% human AB serum, heat-inactivated L-glutamine (2 mM; Valley Biomedical Products and Services, Inc., Winchester, VA), and 2.5 mL penicillin/gentamicin (10 mg/mL) in a tissue culture flask (37°C/5% CO₂). The resultant population is characterized for purity by using flow cytometry. Isolated cells are subsequently cultured with anti-CD3/anti-CD28 monoclonal antibody (mAb)-coated Dynabeads (Invitrogen) at a 3:1 bead to cell ratio for 14 ± 1 days. On day 0, cultures are supplemented with 200 IU/mL IL-2 (Proleukin, Chiron Corporation, Emeryville, CA). Cells are maintained at a density of 1.0 x 10⁶ viable nucleated cells/mL by splitting every 48 - 72 hours for 14 days until harvesting. All products that pass lot release criteria include: 7AAD viability ≥70%, CD4+CD25+ purity ≥60%, less than 10% CD4-/CD8+ cells, anti-CD3/anti-CD28 mAB bead count <100 per 3 x 10⁶ cells, gram stain with 'no organisms', and endotoxin <5 EU/kg. Fucosylation is conducted using FTVI at a concentration shown to be optimal for cGMP expanded T_{regs} plus GDP-fucose at 1 mM for 30 minutes at room temperature. A portion of the cells is suspended in RPMI 1640 supplemented with pyruvate (0.02 mM), penicillin (100 U/mL), streptomycin (100 mg/ mL), 20% human pooled serum (HPS), and 15% dimethylsulfoxide, and cryopreserved using a controlled-rate freezer followed by transfer to the vapor phase of a liquid nitrogen tank. After two weeks the cells are quickly thawed in a 37°C water bath and washed twice before use. Thawed cells are tested for fucosylation levels two hours following thawing, gating on viable cells using 7AAD staining. Fucosylation levels (as measured by MFI) should be observed to be ±10% of levels observed prior to cryopreservation.

### Example 11

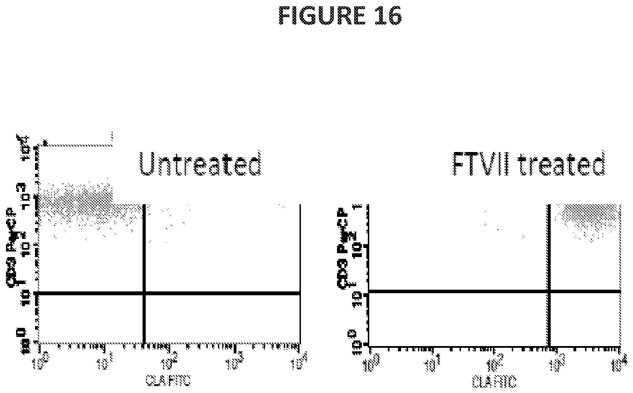

Cytotoxic T cells were expanded against CG1 peptide (amino acid sequence FLLPTGAEA; SEQ ID NO:1) that binds HLA-A2. Dendritic cells (DC) were generated from HLA-A*0201 healthy donor monocytes by adherence and immunostimulation and then co-cultured with PBMC from the same healthy donor. After an adherence step at 37°C, cells remaining in suspension (lymphocytes) were removed and pulsed with 40 µg/mL of CG1 peptide followed by stimulation with IL-7 (10 ng/mL) and IL-2 (10 ng/mL) for 5 days. Adherent cells from the initial step were matured into monocyte-derived DC by addition of GM-CSF (100 ng/mL), IL-4 (50 ng/mL), and TNF-α (25 ng/mL). After 5 days, DC were detached and pulsed with appropriate peptides at 40 µg/mL and subsequently combined with the remainder of autologous lymphocyte population. Co-cultures were then re-stimulated with IL-7 (10 ng/mL) and IL-2 (25 ng/mL) for 7 days to allow for CTL proliferation. On day 12, cells were harvested and analyzed by dextramer staining and *in vitro* cytotoxicity assays to confirm CTL expansion and specificity. The cells were then either fucosylated with TZ102 (1 mM GDP-fucose plus 75 µg/mL FTVII) by incubation at room temperature for 30 minutes and then washed ("FTVII treated") or given a mock incubation in the absence of FTVII enzyme ("untreated"). Fucosylation levels were determined by flow cytometry using the HECA-452 antibody (BD Biosciences) autologous lymphocyte population. Co-cultures were then re-stimulated with IL-7 (10 ng/mL) and IL-2 (25 ng/mL) for 7 days to allow for CTL proliferation. On day 12, cells were harvested and analyzed by dextramer staining and *in vitro* cytotoxicity assays to confirm CTL expansion and specificity. The cells were then either fucosylated with TZ102 (1 mM GDP-fucose plus 75 µg/mL FTVII) by incubation at room temperature for 30 minutes and then washed ("FTVII treated") or given a mock incubation in the absence of FTVII enzyme ("untreated"). Fucosylation levels were determined by flow cytometry using the anti-CLA-FITC (HECA-452) antibody (BD Biosciences). As can be seen in Figure 16, virtually 100% of the cells were fucosylated by treatment with TZ102.

### Example 12

Cytotoxic T cells are prepared and fucosylated under cGMP conditions using the methodology described in Example 11. All reagents are cGMP grade including CG1 peptide and FTVII. Fucosylation levels are measured using anti-CLA-FITC as described in Example 11. A portion of the cells is suspended in RPMI 1640 supplemented with pyruvate (0.02 mM), penicillin (100 U/mL), streptomycin (100 mg/mL), 20% human pooled serum (HPS), and 15% dimethylsulfoxide, and cryopreserved using a controlled-rate freezer followed by transfer to the vapor phase of a liquid nitrogen tank. After two weeks the cells are quickly thawed in a 37°C water bath and washed twice before use. Thawed cells are tested for fucosylation levels two hours following thawing, gating on viable cells using 7AAD staining. Fucosylation levels as measured by MFI should be observed to be ±10% of levels observed prior to cryopreservation.

## Claims

1. A method of cryopreserving fucosylated therapeutic cells, the method comprising the steps of:
isolating therapeutic cells;
fucosylating the therapeutic cells by contacting them with an effective amount of an α1,3-fucosyltransferase and GDP-fucose;
mixing the fucosylated therapeutic cells with a therapeutic cell cryopreservation composition comprising a physiologically balanced salt solution, at least one permeating cryoprotectant, and at least one non-permeating cryoprotectant to form a mixture; and
freezing the mixture by cooling at a rate in a range of from 1°C to 2°C per minute from 37°C to -80°C to produce a frozen cell suspension, and transferring the frozen cell suspension to storage in the presence of liquid nitrogen.

2. The method of claim 1, further comprising the step of expanding the therapeutic cells prior to the fucosylating step.

3. The method of claim 2, wherein the step of expanding the therapeutic cells is further defined as expanding the therapeutic cells under current good manufacturing practice (cGMP) conditions.

4. The method of any one of claims 1-3, wherein the α1,3-fucosyltransferase is selected from the group consisting of an α1,3-fucosyltransferase III, an α1,3-fucosyltransferase IV, an α1,3-fucosyltransferase V, an α1,3-fucosyltransferase VI, an α1,3-fucosyltransferase VII, an α1,3-fucosyltransferase IX, an α1,3-fucosyltransferase X, an α1,3-fucosyltransferase XI, and combinations thereof.

5. The method of any one of claims 1-4, wherein the therapeutic cells are isolated from at least one isolated tissue selected from the group consisting of bone marrow, cord blood, umbilical cord, Wharton's jelly, peripheral blood, lymphoid tissue, endometrium, trophoblast-derived tissues, placenta, amniotic fluid, adipose tissue, muscle, liver, cartilage, nervous tissue, cardiac tissue, dental pulp tissue, exfoliated teeth, and combinations thereof.

6. The method of any one of claims 1-5, wherein the isolated therapeutic cells are at least one of differentiated embryonic stem cells and differentiated induced pluripotent stem cells.

7. The method of any one of claims 1-6, wherein the isolated therapeutic cells are selected from the group consisting of hematopoietic stem cells, immune cells, mesenchymal stem cells, muscle cells, amniotic cells, endometrial cells, neural stem cells, natural killer (NK) cells, T cells, B cells, and combinations thereof.

8. The method of any one of claims 1-7, wherein the α1,3-fucosyltransferase is α1,3-fucosyltransferase VII, and the isolated therapeutic cells are selected from the group consisting of T cells, NK cells, B cells, neural stem cells, and combinations thereof.

9. The method of any one of claims 1-8, wherein the physiologically balanced salt solution is the tissue culture medium in which the cells are expanded.

10. The method of any one of claims 1-9, wherein the physiologically balanced salt solution contains protein.

11. The method of claim 10, wherein the protein is selected from the group consisting of fetal bovine serum, horse serum, human serum, human platelet lysate, bovine albumin, human albumin, and combinations thereof.

12. The method of any one of claims 1-11, wherein the at least one permeating cryoprotectant is selected from the group consisting of dimethyl sulfoxide, glycerol, sucrose, ethylene glycol, 1,2-propanediol, and combinations thereof, and wherein the at least one non-permeating cryoprotectant is selected from the group consisting of hydroxyethyl starch, albumin, sucrose, trehalose, dextrose, polyvinyl pyrrolidone, and combinations thereof.

## Patentansprüche

1. Verfahren zur Kryokonservierung von fucosylierten therapeutischen Zellen, wobei das Verfahren die folgenden Schritte umfasst:
Isolieren therapeutischer Zellen;
Fucosylierung der therapeutischen Zellen durch Inkontaktbringen dieser mit einer wirksamen Menge einer α1,3-Fucosyltransferase und GDP-Fucose;
Mischen der fucosylierten therapeutischen Zellen mit einer therapeutischen Zell-Kryokonservierungszusammensetzung, die eine physiologisch ausgewogene Salzlösung, mindestens ein permeierendes Kryoschutzmittel und mindestens ein nichtpermeierendes Kryoschutzmittel umfasst, um ein Gemisch zu bilden; und
Einfrieren des Gemisches durch Abkühlen mit einer Geschwindigkeit im Bereich von 1 °C bis 2 °C pro Minute von 37 °C auf -80 °C zur Herstellung einer gefrorenen Zellsuspension und Überführen der gefrorenen Zellsuspension zur Lagerung in Gegenwart von flüssigem Stickstoff.

2. Verfahren nach Anspruch 1, ferner umfassend den Schritt des Expandierens der therapeutischen Zellen vor dem Fucosylierungsschritt.

3. Verfahren nach Anspruch 2, wobei der Schritt des Expandierens der therapeutischen Zellen ferner als Expandieren der therapeutischen Zellen unter Bedingungen der aktuellen Guten Herstellungspraxis (current Good Manufacturing Practice, cGMP) definiert ist.

4. Verfahren nach einem der Ansprüche 1-3, wobei die α1,3-Fucosyltransferase ausgewählt ist aus der Gruppe bestehend aus einer α1,3-Fucosyltransferase III, einer α1,3-Fucosyltransferase IV, einer α1,3-Fucosyltransferase V, einer α1,3-Fucosyltransferase VI, einer α1,3-Fucosyltransferase VII, einer α1,3-Fucosyltransferase IX, einer α1,3-Fucosyltransferase X, einer α1,3-Fucosyltransferase XI und Kombinationen davon.

5. Verfahren nach einem der Ansprüche 1-4, wobei die therapeutischen Zellen aus mindestens einem isolierten Gewebe isoliert werden, das ausgewählt ist aus der Gruppe bestehend aus Knochenmark, Nabelschnurblut, Nabelschnur, Wharton-Sulze, peripherem Blut, Lymphoidgewebe, Endometrium, von Trophoblasten abgeleiteten Geweben, Plazenta, Fruchtwasser, Fettgewebe, Muskel, Leber, Knorpel, Nervengewebe, Herzgewebe, Zahnpulpa-Gewebe, exfoliierten Zähnen und Kombinationen davon.

6. Verfahren nach einem der Ansprüche 1-5, wobei die isolierten therapeutischen Zellen mindestens eines aus differenzierten embryonalen Stammzellen und differenzierten induzierten pluripotenten Stammzellen sind.

7. Verfahren nach einem der Ansprüche 1-6, wobei die isolierten therapeutischen Zellen ausgewählt sind aus der Gruppe bestehend aus hämatopoetischen Stammzellen, Immunzellen, mesenchymalen Stammzellen, Muskelzellen, amniotischen Zellen, Endometriumzellen, neuralen Stammzellen, NK-Zellen (Natürliche-Killer-Zellen), T-Zellen, B-Zellen und Kombinationen davon.

8. Verfahren nach einem der Ansprüche 1-7, wobei die α1,3-Fucosyltransferase α1,3-Fucosyltransferase VII ist und die isolierten therapeutischen Zellen ausgewählt sind aus der Gruppe bestehend aus T-Zellen, NK-Zellen, B-Zellen, neuralen Stammzellen und Kombinationen davon.

9. Verfahren nach einem der Ansprüche 1-8, wobei die physiologisch ausgewogene Salzlösung das Gewebekulturmedium ist, in dem die Zellen expandiert werden.

10. Verfahren nach einem der Ansprüche 1-9, wobei die physiologisch ausgewogene Salzlösung Protein enthält.

11. Verfahren nach Anspruch 10, wobei das Protein ausgewählt ist aus der Gruppe bestehend aus fötalem Rinderserum, Pferdeserum, Humanserum, humanem Thrombozytenlysat, Rinderalbumin, Humanalbumin und Kombinationen davon.

12. Verfahren nach einem der Ansprüche 1-11, wobei das mindestens eine permeierende Kryoschutzmittel ausgewählt ist aus der Gruppe bestehend aus Dimethylsulfoxid, Glycerin, Saccharose, Ethylenglykol, 1,2-Propandiol, und Kombinationen davon, und wobei das mindestens eine nichtpermeierende Kryoschutzmittel ausgewählt ist aus der Gruppe bestehend aus Hydroxyethylstärke, Albumin, Saccharose, Trehalose, Dextrose, Polyvinylpyrrolidon und Kombinationen davon.

## Revendications

1. Procédé de cryopréservation de cellules thérapeutiques fucosylées, le procédé comprenant les étapes de :
isolement de cellules thérapeutiques ;
fucosylation des cellules thérapeutiques en les mettant en contact avec une quantité efficace d'une α1,3-fucosyltransférase et de GDP-fucose ;
mélange des cellules thérapeutiques fucosylées avec une composition de cryopréservation de cellules thérapeutiques comprenant une solution saline physiologiquement équilibrée, au moins un cryoprotecteur perméant et au moins un cryoprotecteur non perméant pour former un mélange ; et
congélation du mélange par refroidissement à une vitesse dans une plage de 1 °C à 2 °C par minute de 37 °C à -80 °C pour produire une suspension de cellules congelées, et transfert de la suspension de cellules congelées à un stockage en présence d'azote liquide.

2. Procédé selon la revendication 1, comprenant en outre l'étape d'expansion des cellules thérapeutiques avant l'étape de fucosylation.

3. Procédé selon la revendication 2, dans lequel l'étape d'expansion des cellules thérapeutiques est en outre définie comme l'expansion des cellules thérapeutiques dans des conditions de bonnes pratiques de fabrication actuelles (cGMP).

4. Procédé selon l'une quelconque des revendications 1 à 3, dans lequel la α1,3-fucosyltransférase est choisie dans le groupe constitué par une α1,3-fucosyltransférase III, une α1,3-fucosyltransférase IV, une α1,3-fucosyltransférase V, une α1,3-fucosyltransférase VI, une α1,3-fucosyltransférase VII, une α1,3-fucosyltransférase IX, une α1,3-fucosyltransférase X, une α1,3-fucosyltransférase XI et des combinaisons correspondantes.

5. Procédé selon l'une quelconque des revendications 1 à 4, dans lequel les cellules thérapeutiques sont isolées à partir d'au moins un tissu isolé choisi dans le groupe constitué par la moelle osseuse, le sang du cordon, le cordon ombilical, la gelée de Wharton, le sang périphérique, le tissu lymphoïde, l'endomètre, des tissus dérivés du trophoblaste, le placenta, le liquide amniotique, le tissu adipeux, un muscle, le foie, le cartilage, le tissu nerveux, le tissu cardiaque, le tissu pulpaire dentaire, les dents exfoliées et des combinaisons correspondantes.

6. Procédé selon l'une quelconque des revendications 1 à 5, dans lequel les cellules thérapeutiques isolées sont au moins l'une parmi des cellules souches embryonnaires différenciées et des cellules souches pluripotentes induites différenciées.

7. Procédé selon l'une quelconque des revendications 1 à 6, dans lequel les cellules thérapeutiques isolées sont choisies dans le groupe constitué par des cellules souches hématopoïétiques, des cellules immunitaires, des cellules souches mésenchymateuses, des cellules musculaires, des cellules amniotiques, des cellules endométriales, des cellules souches neurales, des cellules tueuses naturelles (NK), des cellules T, des cellules B et des combinaisons correspondantes.

8. Procédé selon l'une quelconque des revendications 1 à 7, dans lequel la α1,3-fucosyltransférase est la α1,3-fucosyltransférase VII, et les cellules thérapeutiques isolées sont choisies dans le groupe constitué par les cellules T, les cellules NK, les cellules B, les cellules souches neurales et des combinaisons correspondantes.

9. Procédé selon l'une quelconque des revendications 1 à 8, dans lequel la solution saline physiologiquement équilibrée est le milieu de culture tissulaire dans lequel les cellules sont expansées.

10. Procédé selon l'une quelconque des revendications 1 à 9, dans lequel la solution saline physiologiquement équilibrée contient une protéine.

11. Procédé selon la revendication 10, dans lequel la protéine est choisie dans le groupe constitué par le sérum fœtal bovin, le sérum de cheval, le sérum humain, le lysat plaquettaire humain, l'albumine bovine, l'albumine humaine et des combinaisons correspondantes.

12. Procédé selon l'une quelconque des revendications 1 à 11, dans lequel l'au moins un cryoprotecteur perméant est choisi dans le groupe constitué par le diméthylsulfoxyde, le glycérol, le saccharose, l'éthylène glycol, le 1,2-propanediol et des combinaisons correspondantes, et dans lequel l'au moins un cryoprotecteur non perméant est choisi dans le groupe constitué par l'hydroxyéthylamidon, l'albumine, le saccharose, le tréhalose, le dextrose, la polyvinylpyrrolidone et des combinaisons correspondantes.
